# EUROPEAN PATENT APPLICATION

(11) **EP 4 487 848 A1**
(43) Date of publication of application: **08.01.2025**
(21) Application number: 23760201.6
(22) Date of filing: 28.02.2023
(51) Int. Cl.: A61K 31/454, A61K 31/4545, A61K 31/496, A61K 31/4995, A61K 31/5377, A61P 13/12

(54) **PHARMACEUTICAL COMPOSITION FOR PREVENTING AND/OR TREATING RENAL DISEASE**

(30) Priority: 28.02.2022 JP 2022029773
(71) Applicant: OSAKA UNIVERSITY, Suita-shi Osaka 565-0871 (JP)
(72) Inventor: NAGATA, Ryu, Suita-shi, Osaka 565-0871 (JP); ISAKA, Yoshitaka, Suita-shi, Osaka 565-0871 (JP); YAMAMOTO, Takeshi, Suita-shi, Osaka 565-0871 (JP); YONISHI, Hiroaki, Suita-shi, Osaka 565-0871 (JP); MORI, Masayuki, Kitakyushu-shi, Fukuoka 807-8555 (JP); SAKAGUCHI, Reiko, Kitakyushu-shi, Fukuoka 807-8555 (JP); OKADA, Ryo, Kitakyushu-shi, Fukuoka 807-8555 (JP)
(74) Representative: Studio Torta S.p.A.
(86) International application number: PCT/JP2023/007230
(87) International publication number: WO 2023/163203

(57) **Abstract**

An object of the invention is to provide a pharmaceutical composition, in particular, an oral pharmaceutical composition useful for, for example, preventing and/or treating a kidney disease, in particular, a disease caused by glomerular injury. The invention relates to a pharmaceutical composition for preventing and/or treating a kidney disease, comprising a compound represented by formula (1), a salt thereof, or a prodrug thereof: 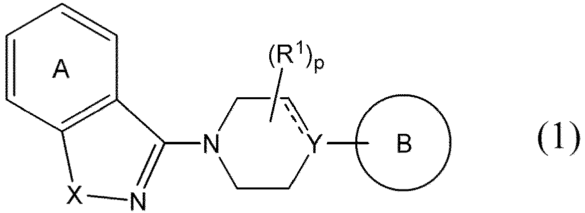 wherein
A is an optionally substituted benzene ring;
B is an optionally substituted aryl or an optionally substituted heteroaryl;
X is an oxygen atom or a sulfur atom;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

## Description

### Technical Field

The present invention relates to, for example, a pharmaceutical composition for preventing and/or treating a kidney disease.

### Background Art

Pathological injury in the kidneys etc. causes severe proteinuria. Causes of proteinuria include acute glomerulonephritis, chronic glomerulonephritis, diabetic nephropathy, and the like. When a large amount of protein is excreted in the urine, the level of blood protein (mainly albumin) decreases, resulting in a condition known as nephrotic syndrome. Further, urinary protein itself causes renal tubular injury or interstitial injury and deteriorates kidney function. Until now, drugs (antihypertensives, steroid agents, immunosuppressive agents, etc.), kidney transplantation, dialysis, and the like have been used for symptomatic therapy to suppress proteinuria; however, there still has been no fundamental therapeutic agent. Furthermore, gene modification therapies for humans typically require a huge cost, and problems still remain in terms of safety verification. Accordingly, development of therapeutic agents that are easily administered are in demand.

### Citation List

### Patent Literature

PTL 1: WO2019/208812

### Summary of Invention

### Technical Problem

An object is to provide a pharmaceutical composition, in particular, an oral pharmaceutical composition useful for, for example, preventing and/or treating a kidney disease, in particular, a disease caused by glomerular injury.

### Solution to Problem

To solve the above problem, the present inventors conducted extensive research and consequently found that a compound represented by formula (1), a compound represented by formula (2), a salt thereof, or a prodrug thereof, described in PTL 1, can attenuate a kidney disease, in particular, chronic glomerulonephritis, and can reduce high urinary protein levels. The present invention has thus been accomplished. Representative inventions of the present invention are as follows.

### Item 1.

A pharmaceutical composition for preventing and/or treating a kidney disease, comprising a compound represented by formula (1), a salt thereof, or a prodrug thereof: 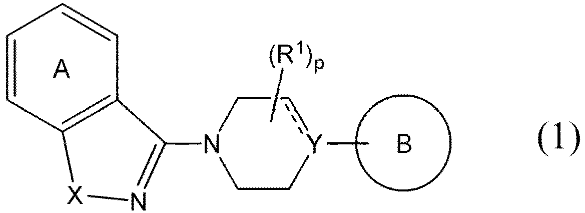 wherein
A is an optionally substituted benzene ring;
B is an optionally substituted aryl or an optionally substituted heteroaryl;
X is an oxygen atom or a sulfur atom;
Y is a nitrogen atom or a carbon atom;
------ of -----Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

### Item 2.

The pharmaceutical composition according to Item 1, wherein in formula (1),
B is an optionally substituted monocyclic aryl or an optionally substituted monocyclic or bicyclic nitrogen-containing heteroaryl.

### Item 3.

The pharmaceutical composition according to Item 1 or 2, wherein in formula (1),
A is a benzene ring optionally substituted with at least one substituent selected from the group consisting of the following A-1 to A-16:
A-1) halogen,
A-2) hydroxyl,
A-3) nitro,
A-4) cyano,
A-5) carboxyl,
A-6) optionally substituted amino,
A-7) optionally substituted cyclic amino,
A-8) optionally substituted lower alkyl,
A-9) optionally substituted lower alkoxy,
A-10) lower alkoxycarbonyl,
A-11) lower alkylsulfonyl,
A-12) carbamoyl optionally substituted with lower alkyl or lower alkylsulfonyl,
A-13) optionally substituted cyclic aminocarbonyl,
A-14) sulfamoyl optionally substituted with lower alkyl,
A-15) optionally substituted cyclic aminosulfonyl, and
A-16) tetrazolyl.

### Item 4.

The pharmaceutical composition according to any one of Items 1 to 3, wherein in formula (1),
B is a monocyclic aryl or a monocyclic or bicyclic heteroaryl, the monocyclic aryl is optionally substituted with at least one substituent selected from the group consisting of the following B-1 to B-16, and
the monocyclic or bicyclic heteroaryl is optionally substituted with at least one substituent selected from the group consisting of the following B-1 to B-17:
   B-1) halogen,
   B-2) hydroxyl,
   B-3) nitro,
   B-4) cyano,
   B-5) carboxyl
   B-6) optionally substituted amino,
   B-7) optionally substituted cyclic amino,
   B-8) optionally substituted lower alkyl,
   B-9) optionally substituted lower alkoxy,
   B-10) lower alkoxycarbonyl,
   B-11) lower alkylsulfonyl,
   B-12) carbamoyl optionally substituted with lower alkyl or lower alkylsulfonyl,
   B-13) optionally substituted cyclic aminocarbonyl,
   B-14) sulfamoyl optionally substituted with lower alkyl,
   B-15) optionally substituted cyclic aminosulfonyl,
   B-16) tetrazolyl, and
   B-17) oxo.

### Item 5.

The pharmaceutical composition according to any one of Items 1 to 4, wherein in formula (1), the benzisoxazole or benzisothiazole skeleton is substituted at the 4-position.

### Item 6.

The pharmaceutical composition according to any one of Items 1 to 3, wherein in formula (1),
B is substituted pyridyl or substituted phenyl, and at least one of the carbon atoms in ortho-positions with respect to the Y-bound carbon atom on the pyridine or benzene ring is substituted.

### Item 7.

The pharmaceutical composition according to any one of Items 1 to 4, wherein in formula (1),
A is a benzene ring optionally substituted with at least one substituent selected from the group consisting of halogen, lower alkoxy, and optionally halogen-substituted lower alkyl;
B is pyridyl or phenyl each optionally substituted with at least one substituent selected from the group consisting of the following B-1, B-5, B-8, B-10, B-12, and B-13:
   B-1) halogen,
   B-5) carboxyl,
   B-8) optionally substituted lower alkyl,
   B-10) lower alkoxycarbonyl,
   B-12) carbamoyl optionally substituted with lower alkyl or lower alkylsulfonyl, and
   B-13) optionally substituted cyclic aminocarbonyl; and
   each R¹ independently represents C₁-C₃ alkyl, or
   two R¹s are bound to each other to form a methylene group, a dimethylene group, or a trimethylene group, or
   (R¹)ₚ is oxo.

### Item 8.

The pharmaceutical composition according to any one of Items 1 to 4 and 7, wherein the compound represented by formula (1) is a compound represented by formula (1A): 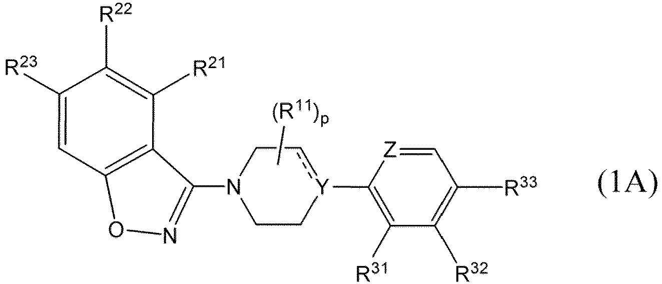 wherein
Z is a nitrogen atom or CH;
Y is a nitrogen atom or a carbon atom;
------ of ------Y is a single or double bond when Y is a carbon atom, or ------ of -----Y is a single bond when Y is a nitrogen atom;
each R¹¹ independently represents methyl or ethyl, or two R¹¹s may be bound to each other to form a crosslinked structure by methylene, dimethylene, or trimethylene;
p is 0, 1, or 2; or
(R¹¹)ₚ is oxo;
R²¹, R²², and R²³ independently represent a hydrogen atom, halogen, carbamoyl, or trifluoromethyl; and
R³¹, R³², and R³³ independently represent a hydrogen atom, halogen, halogen-substituted lower alkyl, methyl, carboxyl, lower alkoxycarbonyl, monomethylaminocarbonyl, or
dimethylaminocarbonyl.

### Item 9.

The pharmaceutical composition according to Item 8, wherein in formula (1A),
R²¹ is a chlorine atom or trifluoromethyl,
R²² and R²³ are each a hydrogen atom,
R³¹ is a chlorine atom,
R³² is a hydrogen atom, and
R³³ is a hydrogen atom, carboxyl, or lower alkoxycarbonyl.

### Item 10.

The pharmaceutical composition according to any one of Items 1 to 4, wherein the compound is Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 221, Compound 281, Compound 311, Compound 321, Compound 331, Compound 341, Compound 351, Compound 361, Compound 371, Compound 381, Compound 391, Compound 401, Compound 431, or Compound 441, each represented by any of the following structures:

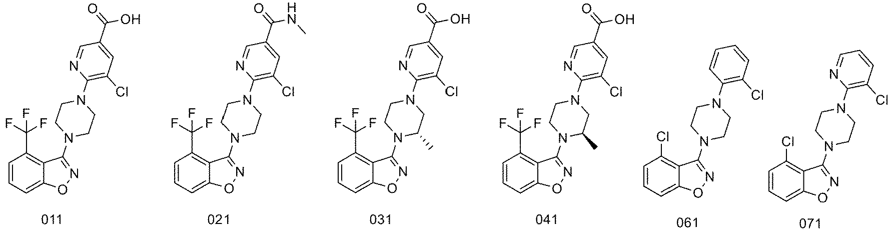

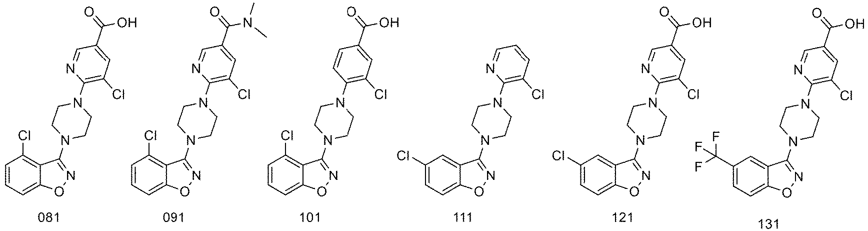

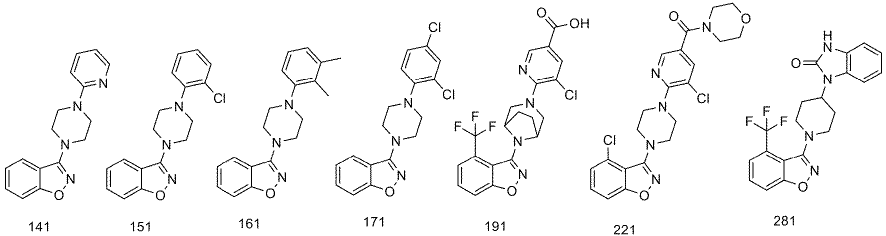

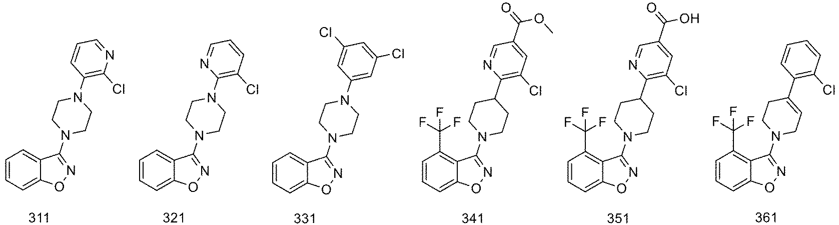

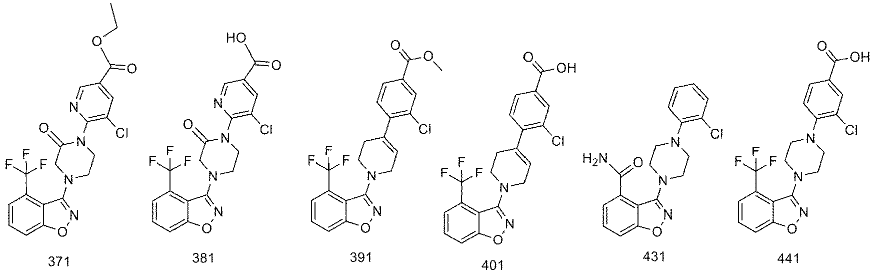

### Item 11.

A pharmaceutical composition for preventing and/or treating a kidney disease, comprising a compound represented by formula (2), a salt thereof, or a prodrug thereof: 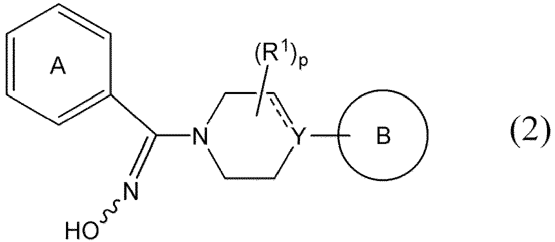 wherein
A is an optionally substituted benzene ring;
B is an optionally substituted aryl or an optionally substituted heteroaryl;
Y is a nitrogen atom or a carbon atom;
------ of ------Y is a single or double bond when Y is a carbon atom, or
------ of ------Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

### Item 12.

The pharmaceutical composition according to any one of Items 1 to 11, wherein the kidney disease is a glomerular disease.

### Item 13.

The pharmaceutical composition according to Item 12, wherein the glomerular disease is chronic glomerulonephritis.

### Item 14.

The pharmaceutical composition according to Item 12, wherein the glomerular disease is diabetic nephropathy.

### Item 15.

The pharmaceutical composition according to any one of Items 12 to 14, wherein the glomerular disease is a disease accompanied by proteinuria.

### Item 16.

A pharmaceutical composition according to any one of Items 1 to 15, which is for oral administration.

### Advantageous Effects of Invention

A compound represented by formula (1), a compound represented by formula (2), a salt thereof, or a prodrug thereof has an action of attenuating a kidney disease, in particular, chronic glomerulonephritis, an action of suppressing a high urinary protein level, an action of suppressing a reduction in serum albumin, or an action of attenuating lymphocyte infiltration and tertiary lymphoid tissue formation.

### Brief Description of Drawings

Fig. 1 is a graph showing urinary protein levels measured in Test Example 1.
Fig. 2 is a graph showing serum albumin levels measured in Test Example 1.
Fig. 3 is a graph showing the left kidney weight per rat body weight measured in Test Example 1.
Fig. 4 is a graph showing the left and right kidney weights of rats measured in Test Example 3.
Fig. 5 is a graph showing the results of PAS staining measured in Test Example 3.
Fig. 6 is a graph showing the results of quantitative PCR measured in Test Example 3.
Fig. 7 is photographs of infiltrated inflammatory cell populations observed in Test Example 3.

### Description of Embodiments

One embodiment of the present invention is directed to a pharmaceutical composition for preventing and/or treating a kidney disease, comprising a compound represented by the following formula (1), a salt thereof, or a prodrug thereof. Another embodiment is directed to a composition for attenuating the progression of a kidney disease, comprising a compound represented by the following formula (1), a salt thereof, or a prodrug thereof. Still another embodiment is directed to a pharmaceutical composition for suppressing deterioration of and/or ameliorating kidney function (in particular, glomerular function), comprising a compound represented by the following formula (1), a salt thereof, or a prodrug thereof.

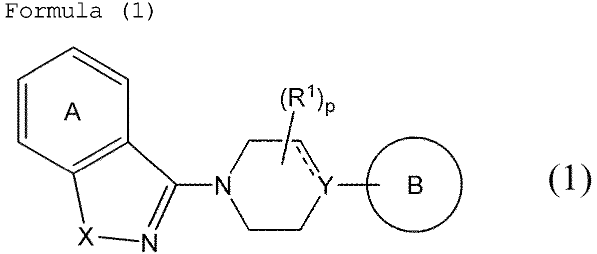

(wherein A is an optionally substituted benzene ring;
B is an optionally substituted aryl or an optionally substituted heteroaryl;
X is an oxygen atom or a sulfur atom;
Y is a nitrogen atom or a carbon atom;
------ of ------Y is a single or double bond when Y is a carbon atom, or
------ of -----Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo) .

In the present invention, examples of substituents for the "optionally substituted benzene ring" include halogen; hydroxyl; nitro; cyano; carboxyl; optionally substituted amino; optionally substituted cyclic amino; optionally substituted lower alkyl; optionally substituted lower alkoxy; lower alkoxycarbonyl, lower alkylsulfonyl; carbamoyl optionally substituted with lower alkyl or lower alkylsulfonyl; optionally substituted cyclic aminocarbonyl; sulfamoyl optionally substituted with lower alkyl; optionally substituted cyclic aminosulfonyl; tetrazolyl; and the like. Such substituents may be used singly or in a combination of two or more.

In the present invention, "aryl" may be, for example, a monocyclic or bicyclic aryl. Specific examples include phenyl, naphthyl, and the like.

In the present invention, the aryl in the "optionally substituted aryl" is as defined above. Examples of substituents for the optionally substituted aryl include halogen; hydroxyl; nitro; cyano; carboxyl; optionally substituted amino; optionally substituted cyclic amino; optionally substituted lower alkyl; optionally substituted lower alkoxy; lower alkoxycarbonyl; lower alkylsulfonyl; carbamoyl optionally substituted with lower alkyl or lower alkylsulfonyl; optionally substituted cyclic aminocarbonyl; sulfamoyl optionally substituted with lower alkyl; optionally substituted cyclic aminosulfonyl; tetrazolyl; oxo; and the like. Such substituents may be used singly or in a combination of two or more.

In the present invention, "oxo" is a group represented by "=O".

In the present invention, "heteroaryl" is, for example, a monocyclic or bicyclic nitrogen-containing heteroaryl. Specific examples include a monocyclic or bicyclic nitrogen-containing heteroaryl that contains one or more (for example, one to three, one or two, or one) nitrogen atoms on the ring and optionally further contains one or more (for example, one to three, one or two, or one) sulfur atoms or oxygen atoms as other heteroatoms. Specific examples of the heteroaryl include pyrrolyl, imidazolyl, triazolyl, tetrazolyl, pyrazolyl, pyridyl, pyrazinyl, pyrimidinyl, pyridazinyl, furyl, thienyl, oxazolyl, isoxazolyl, thiazolyl, isothiazolyl, furazanyl, oxadiazolyl, thiadiazolyl, indolyl, isoindolyl, benzofuranyl, isobenzofuranyl, benzothienyl, isobenzothienyl, indazolyl, quinolyl, isoquinolyl, purinyl, phthalazinyl, pteridyl, naphthyridinyl, quinoxalinyl, quinazolinyl, cinnolinyl, benzoxazolyl, benzothiazolyl, benzimidazolyl, benzofurazanyl, benzothiadiazolyl, benzotriazolyl, isoxazolo[4,5-d]pyridazyl, benzisoxazolyl, benzisothiazolyl, and the like. Preferred is a monocyclic nitrogen-containing heteroaryl or benzimidazolyl.

In the present invention, the heteroaryl in the "optionally substituted heteroaryl" is as defined above. Examples of substituents for the optionally substituted heteroaryl include halogen; hydroxyl; nitro; cyano; carboxyl; optionally substituted amino; optionally substituted cyclic amino; optionally substituted lower alkyl; optionally substituted lower alkoxy; lower alkoxycarbonyl; lower alkylsulfonyl; carbamoyl optionally substituted with lower alkyl or lower alkylsulfonyl; optionally substituted cyclic aminocarbonyl; sulfamoyl optionally substituted with lower alkyl; optionally substituted cyclic aminosulfonyl; tetrazolyl; oxo; and the like. Such substituents may be used singly or in a combination of two or more.

In the present invention, "lower alkyl" includes, for example, C₁-C₈ alkyl, preferably C₁-C₆ alkyl, more preferably C₁-C₄ alkyl, and particularly preferably C₁-C₃ alkyl, each containing a linear, branched, or cyclic structure. Specific examples of linear or branched lower alkyl include methyl, ethyl, 1-propyl, 2-propyl, 1-butyl, 2-butyl, isobutyl, t-butyl, n-pentyl, neopentyl, n-hexyl, isohexyl, 3-methylpentyl, and the like. Examples of lower alkyl containing a cyclic structure include cyclopropyl, cyclopropylmethyl, cyclobutyl, cyclobutylmethyl, cyclopentyl, cyclopentylmethyl, cyclohexyl, cyclohexylmethyl, cyclohexylethyl, and the like. Preferable examples include methyl, ethyl, 2-propyl, t-butyl, cyclopropyl, and the like.

In the present invention, examples of "halogen" include a fluorine atom, a chlorine atom, a bromine atom, an iodine atom, and the like. Preferable examples include a fluorine atom and a chlorine atom.

In the present invention, "optionally substituted amino" refers to an optionally substituted acyclic amino group. Examples of substituents for the amino group include lower alkyl (e.g., methyl, ethyl, and propyl), C₁-C₈ acyl (e.g., acetyl and propionyl), aryl (e.g., phenyl), and heteroaryl. Such substituents may be used singly or in a combination of two or more. Preferable examples of the optionally substituted amino include amino, methylamino, dimethylamino, ethylamino, diethylamino, cyclohexylamino, acetylamino, benzoylamino, phenylamino, and the like.

In the present invention, "cyclic amino" may be, for example, a 5- to 7-membered cyclic amino group containing a nitrogen atom as a ring-constituting atom and optionally further containing one or more (for example, one to three, one or two, or one) oxygen atoms. Specific examples include pyrrolidino, piperidino, piperazino, morpholino, and the like. Preferable examples include pyrrolidino, morpholino, and the like.

In the present invention, the cyclic amino in the "optionally substituted cyclic amino" is as defined above. Examples of substituents for the cyclic amino include lower alkyl, lower alkoxy, amino, hydroxyl, nitro, cyano, carboxyl, oxo, and the like. The cyclic amino may be substituted with at least one substituent selected from the group consisting of the substituents mentioned above. The number of substituents is, for example, 0, 1, 2, or 3, and preferably 0, 1, or 2. Specific examples of the optionally substituted cyclic amino include pyrrolidino, piperidino, piperazino, 4-methylpiperidino, morpholino, 2-pyrrolidonyl, and the like. Preferable examples include pyrrolidino, morpholino, and the like.

In the present invention, the lower alkyl in the "optionally substituted lower alkyl" is as defined above. Examples of substituents for the lower alkyl include hydroxyl; amino; C₁-C₈ alkylamino (e.g., methylamino, ethylamino, propylamino, and t-butylamino); C₁-C₈ alkoxy (e.g., methoxy, ethoxy, 1-propyloxy, 2-propyloxy, and t-butyloxy); halogen (e.g., a fluorine atom, a chlorine atom, and a bromine atom); halo-C₁-C₈ alkoxy (e.g., trifluoromethoxy); aliphatic heterocyclic groups (e.g., morpholino, piperidinyl, pyrrolidinyl, and 4-methyl-1-piperazino); aryl (e.g., phenyl and 1-naphthyl); heteroaryl (e.g., pyridyl, thienyl, and furanyl); carboxyl; C₁-C₈ alkoxycarbonyl (e.g., methoxycarbonyl, ethoxycarbonyl, 1-propoxycarbonyl, 2-propoxycarbonyl, and t-butoxycarbonyl); carbamoyl optionally substituted with lower alkyl (e.g., carbamoyl, methylaminocarbonyl, dimethylaminocarbonyl, and diethylaminocarbonyl); cyclic aminocarbonyl (e.g., pyrrolidinocarbonyl, piperidinocarbonyl, and morpholinocarbonyl); and the like. Examples of preferable substituents include methylamino, ethylamino, dimethylamino, diethylamino, methoxy, ethoxy, 2-propyloxy, t-butoxycarbonyl, hydroxyl, a fluorine atom, a chlorine atom, trichloromethyl, trifluoromethyl, trifluoromethoxy, morpholino, piperidino, pyrrolidino, carboxyl, methoxycarbonyl, ethoxycarbonyl, morpholinocarbonyl, phenyl, pyridyl, and the like. The optionally substituted lower alkyl may be substituted with at least one substituent selected from the group consisting of the substituents mentioned above. The number of substituents may be, for example, 0, 1, 2, or 3, and preferably 0, 1, or 2.

In the present invention, "halogen-substituted lower alkyl" means that all the hydrogen atoms in the alkyl are replaced by halogens. The halogens and lower alkyl in the halogen-substituted lower alkyl are as defined above. The halogens with which the alkyl is substituted are preferably the same. The halogen-substituted lower alkyl is preferably trichloromethyl or trifluoromethyl, and more preferably trifluoromethyl.

In the present invention, examples of "lower alkoxy" include C₁-C₈ alkoxy, preferably C₁-C₆ alkoxy, more preferably C₁-C₄ alkoxy, and particularly preferably C₁-C₃ alkoxy, each containing a linear, branched, or cyclic structure. Specific examples of linear or branched alkoxy include methoxy, ethoxy, 1-propoxy, 2-propoxy, 1-butoxy, 2-butoxy, isobutoxy, t-butoxy, n-pentyloxy, neopentyloxy, n-hexyloxy, isohexyloxy, 3-methylpentyloxy, and the like. Examples of alkoxy containing a cyclic structure include cyclopropoxy, cyclopropylmethoxy, cyclobutyloxy, cyclobutylmethoxy, cyclopentyloxy, cyclopentylmethoxy, cyclohexyloxy, cyclohexylmethoxy, cyclohexylethoxy, and the like. Preferable examples include methoxy, ethoxy, 2-propoxy, t-butoxy, cyclopropoxy, and the like.

In the present invention, the lower alkoxy in the "optionally substituted lower alkoxy" is as defined above. Examples of substituents for the lower alkoxy include hydroxyl; amino; C₁-C₈ alkylamino (e.g., methylamino, ethylamino, propylamino, and t-butylamino); C₁-C₈ alkoxy (e.g., methoxy, ethoxy, 1-propyloxy, 2-propyloxy, and t-butoxy); halogen (e.g., a fluorine atom, a chlorine atom, and a bromine atom); halo-C₁-C₈ alkoxy (e.g., trifluoromethoxy); aliphatic heterocyclic groups (e.g., morpholino, piperidinyl, pyrrolidinyl, and 4-methyl-1-piperazino); aryl (e.g., phenyl and 1-naphthyl); heteroaryl (e.g., pyridyl, thienyl, and furanyl); carboxyl; C₁-C₈ alkoxycarbonyl (e.g., methoxycarbonyl, ethoxycarbonyl, 1-propyloxycarbonyl, 2-propyloxycarbonyl, and t-butoxycarbonyl); carbamoyl optionally substituted with lower alkyl (e.g., carbamoyl, methylaminocarbonyl, dimethylaminocarbonyl, and diethylaminocarbonyl); cyclic aminocarbonyl (e.g., pyrrolidinocarbonyl, piperidinocarbonyl, and morpholinocarbonyl); and the like. Examples of preferable substituents include methylamino, ethylamino, dimethylamino, diethylamino, methoxy, ethoxy, 2-propyloxy, t-butoxycarbonyl, hydroxyl, a fluorine atom, a chlorine atom, trifluoro, morpholino, piperidino, pyrrolidino, carboxyl, methoxycarbonyl, morpholinocarbonyl, phenyl, pyridyl, and the like. The optionally substituted lower alkoxy may be substituted with at least one substituent selected from the group consisting of the substituents mentioned above. The number of substituents may be, for example, 0, 1, 2, or 3, and preferably 0, 1, or 2.

In the present invention, the lower alkoxy in the "lower alkoxycarbonyl" is as defined above. The lower alkoxycarbonyl is a group in which lower alkoxy, such as those mentioned above, is bound to carbonyl. Examples of the lower alkoxycarbonyl include C₁-C₈ alkoxycarbonyl containing a linear, branched, or cyclic structure. Specific examples of the linear or branched alkoxycarbonyl include methoxycarbonyl, ethoxycarbonyl, 1-propoxycarbonyl, 2-propoxycarbonyl, 1-butoxycarbonyl, 2-butoxycarbonyl, isobutoxycarbonyl, t-butoxycarbonyl, and the like. Examples of the C₁-C₈ alkoxycarbonyl containing a cyclic structure include cyclopropoxycarbonyl, cyclopropylmethoxycarbonyl, cyclobutyloxycarbonyl, cyclobutylmethoxycarbonyl, cyclopentyloxycarbonyl, cyclopentylmethoxycarbonyl, cyclohexyloxycarbonyl, cyclohexylmethoxycarbonyl, cyclohexylethoxycarbonyl, and the like. Preferable examples of the lower alkoxycarbonyl include methoxycarbonyl, ethoxycarbonyl, 2-propoxycarbonyl, cyclopropoxycarbonyl, and the like.

In the present invention, the lower alkyl in the "lower alkylsulfonyl" is as defined above. The lower alkylsulfonyl is a group in which lower alkyl, such as those mentioned above, is bound to sulfonyl. Examples of the lower alkylsulfonyl include C₁-C₈ alkylsulfonyl containing a linear, branched, or cyclic structure. Specific examples of the linear or branched alkylsulfonyl include methanesulfonyl, ethanesulfonyl, 1-propylsulfonyl, 2-propylsulfonyl, 1-butylsulfonyl, 2-butylsulfonyl, isobutylsulfonyl, t-butylsulfonyl, and the like. Examples of the C₁-C₈ alkylsulfonyl containing a cyclic structure include cyclopropylsulfonyl, cyclopropylmethylsulfonyl, cyclobutylsulfonyl, cyclobutylmethylsulfonyl, cyclopentylsulfonyl, cyclopentylmethylsulfonyl, cyclohexylsulfonyl, cyclohexylmethylsulfonyl, cyclohexylethylsulfonyl, and the like. Preferable examples include methanesulfonyl, ethanesulfonyl, 2-propanesulfonyl, cyclopropanesulfonyl, and the like.

In the present invention, the lower alkyl and lower alkylsulfonyl in the "carbamoyl optionally substituted with lower alkyl or lower alkylsulfonyl" are as defined above. The carbamoyl optionally substituted with lower alkyl or lower alkylsulfonyl includes "carbamoyl optionally substituted with lower alkyl" and "carbamoyl optionally substituted with lower alkylsulfonyl."

The "carbamoyl optionally substituted with lower alkyl" is a group in which one or two lower alkyl groups, such as those mentioned above, may be bound to carbamoyl. When two lower alkyl groups are bound, the lower alkyl groups may be the same or different. Examples of the carbamoyl optionally substituted with lower alkyl include carbamoyl; aminocarbonyl substituted with C₁-C₈ alkyl containing a linear, branched, or cyclic structure; and the like. Specific examples of the carbamoyl optionally substituted with lower alkyl include carbamoyl, methylaminocarbonyl, ethylaminocarbonyl, propylaminocarbonyl, 2-propylaminocarbonyl, dimethylaminocarbonyl, diethylaminocarbonyl, ethylmethylaminocarbonyl, methylpropylaminocarbonyl, dicyclohexylaminocarbonyl, and the like.

The "carbamoyl optionally substituted with lower alkylsulfonyl" is a group in which one or two lower alkylsulfonyl groups, such as those mentioned above, may be bound to carbamoyl. When two lower alkylsulfonyl groups are bound, the lower alkylsulfonyl groups may be the same or different. Examples of the carbamoyl optionally substituted with lower alkylsulfonyl include carbamoyl; aminocarbonyl substituted with C₁-C₈ alkylsulfonyl containing a linear, branched, or cyclic structure; and the like. Examples of the linear or branched C₁-C₈ alkylsulfonylaminocarbonyl include methanesulfonylaminocarbonyl, ethanesulfonylaminocarbonyl, 1-propylsulfonylaminocarbonyl, 2-propylsulfonylaminocarbonyl, 1-butylsulfonylaminocarbonyl, 2-butylsulfonylaminocarbonyl, isobutylsulfonylaminocarbonyl, t-butylsulfonylaminocarbonyl, and the like. Examples of the C₁-C₈ alkylsulfonylaminocarbonyl containing a cyclic structure include cyclopropylsulfonylaminocarbonyl, cyclopropylmethylsulfonylaminocarbonyl, cyclobutylsulfonylaminocarbonyl, cyclobutylmethylsulfonylaminocarbonyl, cyclopentylsulfonylaminocarbonyl, cyclopentylmethylsulfonylaminocarbonyl, cyclohexylsulfonylaminocarbonyl, cyclohexylmethylsulfonylaminocarbonyl, cyclohexylethylsulfonylaminocarbonyl, and the like. Preferable examples of the carbamoyl optionally substituted with lower alkylsulfonyl include carbamoyl, methanesulfonylaminocarbonyl, ethanesulfonylaminocarbonyl, 2-propylsulfonylaminocarbonyl, cyclopropylsulfonylaminocarbonyl, and the like.

In the present invention, the optionally substituted cyclic amino in the "optionally substituted cyclic aminocarbonyl" is as defined above. The optionally substituted cyclic aminocarbonyl is a group in which optionally substituted cyclic amino, such as those mentioned above, is bound to carbonyl. Specific examples of the optionally substituted cyclic aminocarbonyl include pyrrolidinocarbonyl, piperidinocarbonyl, piperazinocarbonyl, 4-methylpiperidino, morpholinocarbonyl, 2-pyrrolidonylcarbonyl, and the like. Preferable examples include pyrrolidinocarbonyl, morpholinocarbonyl, and the like.

In the present invention, the lower alkyl in the "sulfamoyl optionally substituted with lower alkyl" is as defined above. The sulfamoyl optionally substituted with lower alkyl is a group in which one or two lower alkyl groups, such as those mentioned above, may be bound to sulfamoyl. When two lower alkyl groups are bound, the lower alkyl groups may be the same or different. Examples of the sulfamoyl optionally substituted with lower alkyl include sulfamoyl; aminosulfonyl substituted with C₁-C₈ alkyl containing a linear, branched, or cyclic structure; and the like. Specific examples include sulfamoyl, methylaminosulfonyl, ethylaminosulfonyl, propylaminosulfonyl, 2-propylaminosulfonyl, dimethylaminophonyl, diethylaminosulfonyl, ethylmethylaminosulfonyl, methylpropylaminosulfonyl, dicyclohexylaminosulfonyl, and the like.

In the present invention, the optionally substituted cyclic amino in the "optionally substituted cyclic aminosulfonyl" is as defined above. The optionally substituted cyclic aminosulfonyl is a group in which optionally substituted cyclic amino, such as those mentioned above, is bound to sulfonyl. Specific examples of the optionally substituted cyclic aminosulfonyl include pyrrolidinosulfonyl, piperidinosulfonyl, piperazinosulfonyl, 4-methylpiperidinosulfonyl, morpholinosulfonyl, 4-piperidonylsulfonyl, and the like. Preferable examples include pyrrolidinosulfonyl, morpholinosulfonyl, and the like.

In the present invention, when Y is a carbon atom, ------ is a single or double bond. When Y is a nitrogen atom, ------ is a single bond.

In the compound represented by formula (1), A is an optionally substituted benzene ring. Examples of the substituent for A include at least one member selected from the group consisting of the following A-1 to A-16. When two or more substituents are present, the substituents may be the same or different from each other.
A-1) halogen,
A-2) hydroxyl,
A-3) nitro,
A-4) cyano,
A-5) carboxyl,
A-6) optionally substituted amino,
A-7) optionally substituted cyclic amino,
A-8) optionally substituted lower alkyl,
A-9) optionally substituted lower alkoxy,
A-10) lower alkoxycarbonyl,
A-11) lower alkylsulfonyl,
A-12) carbamoyl optionally substituted with lower alkyl or lower alkylsulfonyl,
A-13) optionally substituted cyclic aminocarbonyl,
A-14) sulfamoyl optionally substituted with lower alkyl,
A-15) optionally substituted cyclic aminosulfonyl, and
A-16) tetrazolyl.

The number of substituents for A is, for example, 0 to 5, 0 to 4, 0 to 3, preferably 0, 1, or 2, and more preferably 0 or 1. When two or more substituents are present, the substituents may be the same or different from each other.

Other examples of substituents for A include at least one member selected from the group consisting of the above A-1 and A-3 to A-16; at least one member selected from the group consisting of the above A-1 and A-3 to A-16 excluding methoxy; and the like.

The substituent for A is preferably at least one member selected from the group consisting of halogen; lower alkoxy; carbamoyl optionally substituted with lower alkyl or lower alkylsulfonyl; and optionally halogen-substituted lower alkyl, more preferably at least one member selected from the group consisting of halogen; lower alkoxy; carbamoyl; and optionally halogen-substituted lower alkyl, even more preferably at least one member selected from the group consisting of halogen, methoxy, ethoxy, carbamoyl, fluoromethyl, difluoromethyl, trifluoromethyl, fluoroethyl, difluoroethyl, and trifluoroethyl, still more preferably at least one member selected from the group consisting of halogen, methoxy, ethoxy, carbamoyl, fluoromethyl, difluoromethyl, trifluoromethyl, fluoroethyl, difluoroethyl, and trifluoroethyl, and particularly preferably at least one member selected from the group consisting of a chlorine atom, a fluorine atom, and trifluoromethyl.

In the compound represented by formula (1), the substituent for A may be bound to any of the carbon atoms at the 4-position, 5-position, 6-position, and 7-position of the benzisoxazole or benzisothiazole skeleton. Preferably, the substituent is bound to at least one of the carbon atoms at the 4-position, 5-position, and 6-position, more preferably to carbon atoms at the 4-position and/or 5-position, and particularly preferably to the carbon atom at the 4-position. In the present invention, the position numbers of the atoms constituting the benzisoxazole or benzisothiazole skeleton are as follows.

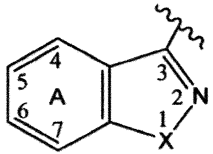

(wherein A and X are as defined above).

In the compound represented by formula (1), particularly preferable A is a benzene ring wherein halogen, lower alkoxy, or optionally halogen-substituted lower alkyl is bound to the carbon atom at the 4-position of the benzisoxazole or benzisothiazole skeleton, and carbon atoms at the 5-, 6-, and 7-positions are not substituted.

In the compound represented by formula (1), B is an optionally substituted aryl or an optionally substituted heteroaryl. The optionally substituted aryl or optionally substituted heteroaryl is as defined above. The aryl is, for example, phenyl or naphthyl, and is preferably phenyl. The heteroaryl is preferably a monocyclic nitrogen-containing heteroaryl that does not contain any other heteroatom as a ring-constituting atom, or benzimidazolyl. The monocyclic nitrogen-containing heteroaryl that does not contain any other heteroatom as a ring-constituting atom is preferably a 5- or 6-membered heteroaryl containing one nitrogen atom as a ring-constituting heteroatom. Examples include pyrrolyl and pyridyl, preferably pyridyl, and more preferably 2-pyridyl. The benzimidazolyl is preferably benzimidazol-3-yl.

When B is a monocyclic aryl, B may be substituted with at least one substituent selected from the group consisting of the following B-1 to B-16. When B is a monocyclic or bicyclic heteroaryl, B may be substituted with at least one substituent selected from the group consisting of the following B-1 to B-17:
B-1) halogen,
B-2) hydroxyl,
B-3) nitro,
B-4) cyano,
B-5) carboxyl,
B-6) optionally substituted amino,
B-7) optionally substituted cyclic amino,
B-8) optionally substituted lower alkyl,
B-9) optionally substituted lower alkoxy,
B-10) lower alkoxycarbonyl,
B-11) lower alkylsulfonyl,
B-12) carbamoyl optionally substituted with lower alkyl or lower alkylsulfonyl,
B-13) optionally substituted cyclic aminocarbonyl,
B-14) sulfamoyl optionally substituted with lower alkyl,
B-15) optionally substituted cyclic aminosulfonyl,
B-16) tetrazolyl, and
B-17) oxo.

The number of substituents for B is, for example, 0 or at least 1, 0 to 5, 0 to 4, preferably 0 to 3, and more preferably 0, 1, or 2. When two or more substituents are present, the substituents may be the same or different from each other.

The substituent for B is preferably, for example, at least one member selected from the group consisting of halogen; carboxyl; optionally substituted lower alkyl; lower alkoxycarbonyl; carbamoyl optionally substituted with lower alkyl or lower alkylsulfonyl; and optionally substituted cyclic aminocarbonyl. Specific examples include at least one member selected from the group consisting of halogen, carboxyl, methyl, ethyl, 1-propyl, 2-propyl, hydroxymethyl, carboxymethyl, trichloromethyl, trifluoromethyl, methoxycarbonyl, ethoxycarbonyl, t-butoxycarbonyl, methylaminocarbonyl, dimethylaminocarbonyl, ethylmethylaminocarbonyl, methanesulfonylaminocarbonyl, pyrrolidinocarbonyl, and morpholinocarbonyl.

The substituent for B is preferably, for example, at least one member selected from the group consisting of halogen; carboxyl; lower alkyl; halogen-substituted lower alkyl; lower alkoxycarbonyl; and carbamoyl optionally substituted with lower alkyl. Specific examples include at least one member selected from the group consisting of halogen, carboxyl, methyl, ethyl, trichloromethyl, trifluoromethyl, methoxycarbonyl, ethoxycarbonyl, n-propoxycarbonyl, isopropoxycarbonyl, monomethylaminocarbonyl, and dimethylaminocarbonyl.

Particularly preferable examples of substituents for B include at least one member selected from the group consisting of a chlorine atom, a fluorine atom, methyl, carboxyl, methoxycarbonyl, ethoxycarbonyl, methylaminocarbonyl, and dimethylaminocarbonyl.

In the compound represented by formula (1), when Y is a nitrogen atom, B is preferably an optionally substituted phenyl or an optionally substituted pyridyl. When Y is a carbon atom, B is preferably an optionally substituted phenyl, an optionally substituted pyridyl, or 2-oxobenzimidazol-3-yl, and more preferably an optionally substituted phenyl or an optionally substituted pyridyl.

In the compound represented by formula (1), when B is substituted pyridyl or substituted phenyl, one or two carbon atoms, preferably one carbon atom, in the ortho-positions with respect to the Y-bound carbon atom on the pyridine or benzene ring are preferably substituted. The substituent(s) bound to the carbon atom(s) in the ortho-position(s) may be any substituent for B, mentioned above. The substituent is preferably halogen, more preferably a chlorine atom or a fluorine atom, and even more preferably a chlorine atom.

In the compound represented by formula (1), when B is substituted pyridyl or substituted phenyl, the carbon atom in the para-position with respect to the Y-bound carbon atom on the pyridine or benzene ring is preferably unsubstituted or substituted with carboxyl.

Further, in the compound represented by formula (1), when B is substituted pyridyl or substituted phenyl, all the carbon atoms in the meta-positions with respect to the Y-bound carbon atom on the pyridine or benzene ring are preferably unsubstituted.

In the compound represented by formula (1), when B is substituted pyridyl or substituted phenyl, it is more preferable that one or two carbon atoms, more preferably one carbon atom, in the ortho-positions with respect to the Y-bound carbon atom on the pyridine or benzene ring are substituted with a chlorine atom or a fluorine atom, that carbon atoms in the meta-positions are unsubstituted, and that the carbon atom in the para-position is unsubstituted or substituted with carboxyl, methoxycarbonyl, or ethoxycarbonyl.

When Y is a nitrogen atom and B is substituted 2-pyridyl, it is particularly preferable that one of the carbon atoms in the ortho-positions with respect to the Y-bound carbon atom on the pyridine ring is substituted with a chlorine atom or a fluorine atom, that all the carbon atoms in the meta-positions are unsubstituted, and that the carbon atom in the para-position is unsubstituted or substituted with carboxyl.

When Y is a nitrogen atom and B is substituted phenyl, it is particularly preferable that one of the two carbon atoms in the ortho-positions with respect to the Y-bound carbon atom on the benzene ring is substituted with a chlorine atom or a fluorine atom, and that all the other carbon atoms that constitute the phenyl are unsubstituted.

When Y is a carbon atom and B is substituted 2-pyridyl, it is particularly preferable that one of the carbon atoms in the ortho-positions with respect to the Y-bound carbon atom on the pyridine ring is substituted with a chlorine atom or a fluorine atom, that all the carbon atoms in the meta-positions are unsubstituted, and that the carbon atom in the para-position is unsubstituted or substituted with carboxyl, methoxycarbonyl, or ethoxycarbonyl.

When Y is a carbon atom and B is substituted phenyl, it is particularly preferable that one of the two carbon atoms in the ortho-positions with respect to the Y-bound carbon atom on the benzene ring is substituted with a chlorine atom or a fluorine atom, that the other one of the carbon atoms in the ortho-positions is unsubstituted, that all the carbon atoms in the meta-positions are unsubstituted, and that the carbon atom in the para-position is unsubstituted or substituted with carboxyl, methoxycarbonyl, or ethoxycarbonyl.

In the compound represented by formula (1), X is an oxygen atom or a sulfur atom, and is preferably an oxygen atom.

In the compound represented by formula (1), Y is a nitrogen atom or a carbon atom, and is preferably a nitrogen atom.

When Y is a carbon atom, ------ is a single or double bond. When Y is a nitrogen atom, ------ is a single bond.

In the compound represented by formula (1), each R¹ independently represents lower alkyl, or two R¹s may be bound to each other to form a spiro ring or a crosslinked structure, or two R¹s may be bound to each other to form a saturated fused heterocycle together with nitrogen and carbon atoms constituting a ring containing Y.

When R¹ is lower alkyl, preferable examples of R¹ include linear or branched C₁-C₃ alkyl. R¹ is more preferably methyl or ethyl, and even more preferably methyl.

When two R¹s are bound to each other to form a spiro ring or a crosslinked structure, forming a spiro ring means that two R¹s are both bound to one of the carbon atoms constituting the ring containing Y in formula (1) and the two R¹s are bound to each other to form a ring together with the carbon atom.

When two R¹s are bound to each other to form a spiro ring or a crosslinked structure, forming a crosslinked structure means that one R¹ group each is bound to two of the carbon atoms constituting the ring containing Y in formula (1) and the R¹s are bound to each other.

Examples of the case in which two R¹s are bound to each other to form a spiro ring or a crosslinked structure include a case in which two R¹s are bound to each other to form methylene, dimethylene, trimethylene, or tetramethylene, thus forming a crosslinked structure, and a case in which two R¹s are bound to each other to form dimethylene or trimethylene, thus forming a spiro ring. A preferable example is a case in which two R¹s are bound to each other to form methylene, dimethylene, or trimethylene, thus forming a crosslinked structure. A crosslinked structure formed by dimethylene, which is represented by the following structural formula, is particularly preferable. 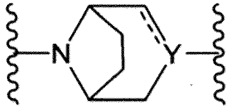 (wherein Y and ------ are as defined above.)

The phrase "two R¹s are bound to each other to form a saturated fused heterocycle together with nitrogen and carbon atoms constituting a ring containing Y" means that one R¹ each is bound to two adjacent carbon atoms among the carbon atoms that constitute a ring containing Y in formula (1) and the R¹s are bound to each other to form a saturated fused heterocycle together with nitrogen and carbon atoms constituting a ring containing Y. The saturated fused heterocycle referred to herein means a fused bicyclic ring of a heterocycle containing Y (a pyrazine ring or a piperidine ring) and a saturated carbon ring containing R¹.

Examples of the saturated fused heterocycle include a fused ring of a pyrazine ring or a piperidine ring and a cyclopentane ring or a cyclohexane ring. Specific examples of the saturated fused heterocycle include octahydrocyclopentapyridine, octahydrocyclopentapyrazine, decahydroquinoline, decahydroquinoxaline, and the like.

R¹ is preferably a crosslinked structure formed by C₁-C₃ alkyl or dimethylene, more preferably a crosslinked structure formed by methyl, ethyl, or dimethylene represented by the above structural formula.

In the compound represented by formula (1), p is 0, 1, or 2.

In the compound represented by formula (1), (R¹)ₚ may be oxo.

Among the compounds represented by formula (1), salts thereof, or prodrugs thereof, a compound represented by the following formula (1A), a salt thereof, or a prodrug thereof is preferable. A pharmaceutical composition for preventing and/or treating a kidney disease, comprising a compound represented by the following formula (1A), a salt thereof, or a prodrug thereof is also included within the scope of the present invention. Another embodiment is directed to a composition for attenuating the progression of a kidney disease, comprising a compound represented by the following formula (1A), a salt thereof, or a prodrug thereof. Still another embodiment is directed to a pharmaceutical composition for suppressing deterioration of and/or ameliorating kidney function, comprising a compound represented by the following formula (1A), a salt thereof, or a prodrug thereof.

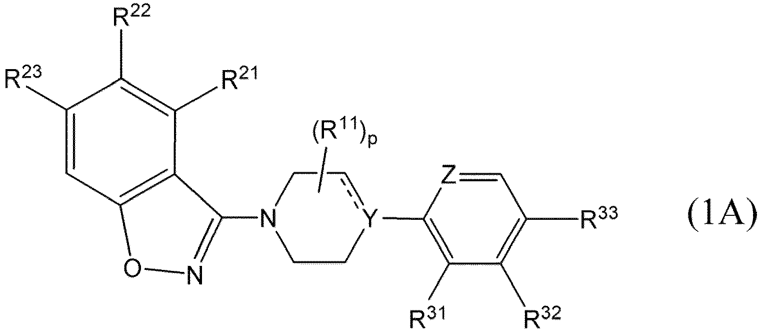

(wherein Z is a nitrogen atom or CH;
Y is a nitrogen atom or a carbon atom;
------ of -----Y is a single or double bond when Y is a carbon atom, or
------ of ------Y is a single bond when Y is a nitrogen atom;
each R¹¹ independently represents methyl or ethyl, or
two R¹¹s may be bound to each other to form a crosslinked structure by methylene, dimethylene, or trimethylene;
p is 0, 1, or 2; or
(R¹¹)ₚ is oxo;
R²¹, R²², and R²³ independently represent a hydrogen atom, halogen, carbamoyl, or trifluoromethyl; and
R³¹, R³², and R³³ independently represent a hydrogen atom, halogen, halogen-substituted lower alkyl, methyl, carboxyl, lower alkoxycarbonyl, monomethylaminocarbonyl, or
dimethylaminocarbonyl).

In the compound represented by formula (1A), Z is a nitrogen atom or CH. When Y is a nitrogen atom, Z is preferably a nitrogen atom.

In the compound represented by formula (1A), Y is a nitrogen atom or a carbon atom.

In the compound represented by formula (1A), each R¹¹ independently represents methyl or ethyl, or two R¹¹s may be bound to each other to form a crosslinked structure by methylene, dimethylene, or trimethylene.

R¹¹ is preferably methyl or ethyl, or a crosslinked structure formed by dimethylene or trimethylene, and more preferably methyl or a crosslinked structure formed by diethylene.

The case in which two R¹¹s are bound to each other to form a crosslinked structure by methylene, dimethylene, or trimethylene refers to a case in which one R¹¹ each is bound to two carbon atoms among the carbon atoms that constitute the ring containing Y in formula (1A) and the R¹¹s are bound to each other to form methylene, dimethylene, or trimethylene, thus forming a crosslinked structure on the piperazine ring.

When the ring containing Y in formula (1A) is substituted with R¹¹, (R¹¹)ₚ is preferably oxo, or the ring is preferably represented by the following structural formula.

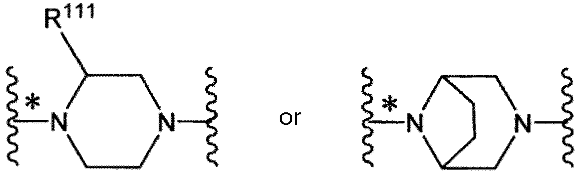

(wherein * represents the side bound to the carbon atom at the 3-position of isobenzoxazole, and R¹¹¹ represents C₁-C₃ alkyl) . R¹¹¹ is preferably methyl or ethyl, and more preferably methyl.

In formula (1A), (R¹¹)ₚ may be oxo.

In the compound represented by formula (1A), R²¹, R²², and R²³ independently represent a hydrogen atom, halogen, carbamoyl, or trifluoromethyl, and at least one of R²¹, R²², and R²³ is preferably halogen, carbamoyl, or trifluoromethyl. R²¹ is preferably a chlorine atom, a fluorine atom, carbamoyl, or trifluoromethyl, and more preferably a chlorine atom or trifluoromethyl. R²² is preferably a hydrogen atom, a chlorine atom, or trifluoromethyl, and more preferably a hydrogen atom. R²³ is preferably a hydrogen atom, a chlorine atom, or trifluoromethyl, and more preferably a hydrogen atom. It is particularly preferable that R²¹ is halogen (preferably a chlorine atom or a fluorine atom) or trifluoromethyl, and that R²² and R²³ are both a hydrogen atom.

In the compound represented by formula (1A), R³¹, R³², and R³³ independently represent a hydrogen atom, halogen, halogen-substituted lower alkyl, methyl, carboxyl, lower alkoxycarbonyl, monomethylaminocarbonyl, or dimethylaminocarbonyl. R³¹ is preferably a hydrogen atom, halogen, trichloromethyl, trifluoromethyl, or methyl, and more preferably halogen, trichloromethyl, trifluoromethyl, or methyl, and particularly preferably a chlorine atom. R³² is preferably a hydrogen atom, halogen, or methyl, and more preferably a hydrogen atom. R³³ is preferably a hydrogen atom, halogen, carboxyl, methoxycarbonyl, ethoxycarbonyl, monomethylaminocarbonyl, or dimethylaminocarbonyl, more preferably a hydrogen atom, carboxyl, methoxycarbonyl, or ethoxycarbonyl, and particularly preferably a hydrogen atom or carboxyl.

For R³¹, R³², and R³³, it is preferable that R³¹ is halogen (preferably a chlorine atom or a fluorine atom), R³² is a hydrogen atom, and R³³ is a hydrogen atom or carboxyl. When R²¹ is halogen (preferably a chlorine atom), it is preferable that R³¹ is halogen (preferably a chlorine atom or a fluorine atom), R³² is a hydrogen atom, and R³³ is a hydrogen atom. When R²¹ is trihalomethyl (preferably trifluoromethyl), it is preferable that R³¹ is halogen (preferably a chlorine atom or a fluorine atom), R³² is a hydrogen atom, and R³³ is a hydrogen atom, carboxyl, methoxycarbonyl, or ethoxycarbonyl. When R²¹ is carbamoyl, it is preferable that R³¹ is halogen (preferably a chlorine atom or a fluorine atom, more preferably a chlorine atom), R³² is a hydrogen atom, and R³³ is a hydrogen atom. It is also preferable that R²¹ is a chlorine atom or trifluoromethyl, R²² and R²³ are both a hydrogen atom, R³¹ is a chlorine atom, R³² is a hydrogen atom, and R³³ is a hydrogen atom or carboxyl.

Specific examples of the compound represented by formula (1), a salt thereof, or a prodrug thereof include Compound 011, Compound 021, Compound 031, Compound 041, Compound 051, Compound 061, Compound 071, Compound 081, Compound 091, Compound 101, Compound 111, Compound 121, Compound 131, Compound 141, Compound 151, Compound 161, Compound 171, Compound 181, Compound 191, Compound 201, Compound 211, Compound 221, Compound 231, Compound 241, Compound 251, Compound 261, Compound 271, Compound 281, Compound 291, Compound 301, Compound 311, Compound 321, Compound 331, Compound 341, Compound 351, Compound 361, Compound 371, Compound 381, Compound 391, Compound 401, Compound 411, Compound 421, Compound 431, and Compound 441. Preferable examples include the following compounds, salts thereof, and prodrugs thereof.

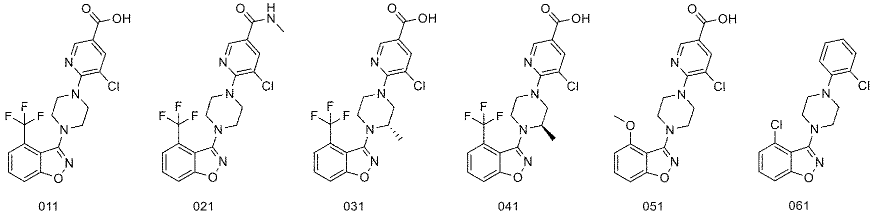

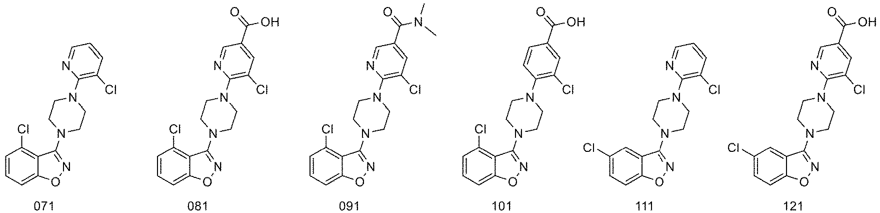

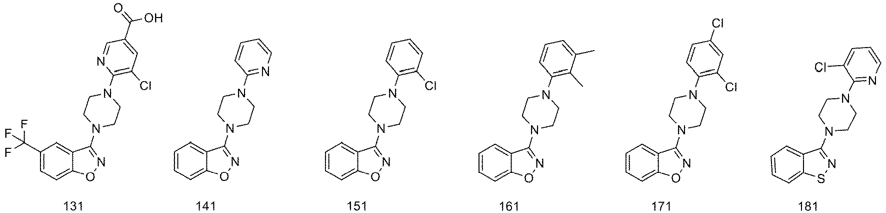

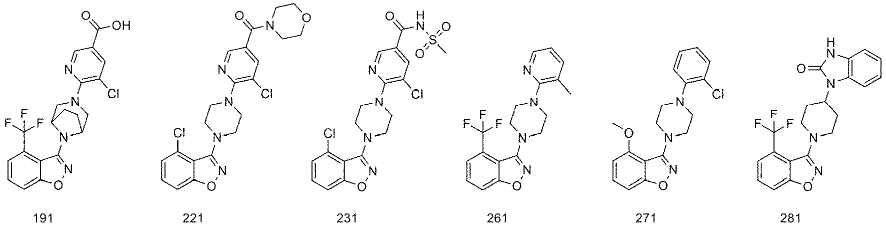

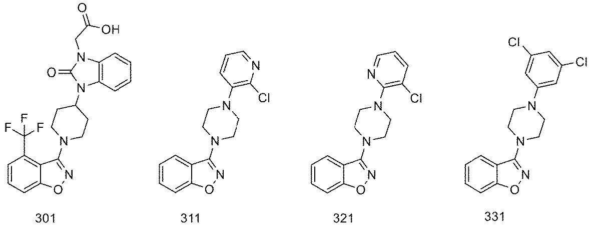

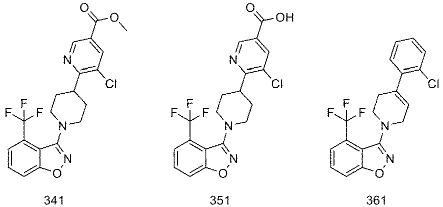

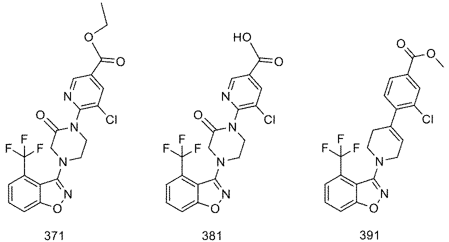

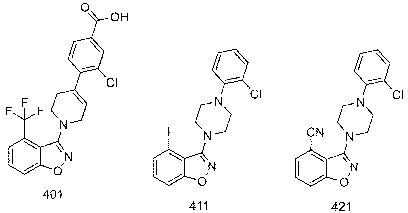

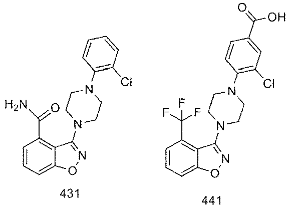

The compound represented by formula (1), a salt thereof, or a prodrug thereof is
more preferably Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 221, Compound 281, Compound 311, Compound 321, Compound 331, Compound 341, Compound 351, Compound 361, Compound 371, Compound 381, Compound 391, Compound 401, Compound 431, or Compound 441; a salt thereof; or a prodrug thereof,
even more preferably Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 321, Compound 351, Compound 361, Compound 371, Compound 381, Compound 401, Compound 431, or Compound 441; a salt thereof; or a prodrug thereof,
still more preferably Compound 011, Compound 031, Compound 041, Compound 061, Compound 071, Compound 191, Compound 361, Compound 371, Compound 381, Compound 401, Compound 431, or Compound 441; a salt thereof; or a prodrug thereof; and
particularly preferably Compound 011, Compound 031, Compound 041, Compound 061, Compound 071, Compound 191, Compound 361, Compound 371, Compound 381, Compound 401, or Compound 441; a salt thereof; or a prodrug thereof.

One embodiment of the present invention includes a pharmaceutical composition for preventing and/or treating a kidney disease, comprising a compound represented by the following formula (2), a salt thereof, or a prodrug thereof. Another embodiment is directed to a composition for attenuating the progression of a kidney disease, comprising a compound represented by the following formula (2), a salt thereof, or a prodrug thereof. Still another embodiment is directed to a pharmaceutical composition for suppressing deterioration of and/or ameliorating kidney function, comprising a compound represented by the following formula (2), a salt thereof, or a prodrug thereof.

Since the compound represented by the following formula (2) is structurally similar to the compound represented by formula (1), the compound represented by the following formula (2), a salt thereof, or a prodrug thereof can have an effect of preventing and/or treating a kidney disease, can have an action of attenuating the progression of a kidney disease, and can have an action of suppressing deterioration of and/or ameliorating kidney function. Furthermore, the compound or a salt thereof can also be used as an intermediate compound for the compound represented by formula (1).

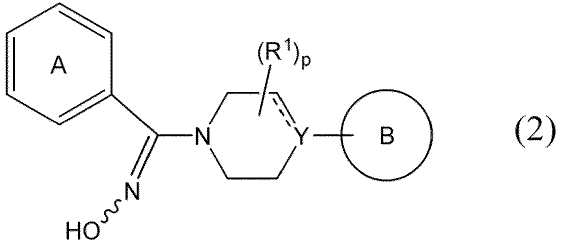

(wherein A, B, Y, R¹, p, and ------ are as defined above) .

In the compound represented by formula (2), A, B, Y, R¹, p, and ------ are as defined above.

In formula (2), it is preferable that one or two of the carbon atoms in the ortho-positions with respect to the A-ring (benzene-ring) carbon atom that is bound to the carbon atom constituting the oxime structure are substituted. For the substituents here, the description above for the substituents for the A ring (benzene ring) in formula (1) may be applied, unless otherwise stated. Thus, the substituents may be, for example, at least one substituent selected from the group consisting of A-1 to A-16 above.

Further, in formula (2), the bond between the nitrogen atom and the hydroxyl group is represented by a wavy line, which is a line indicated below. This line indicates that the compound represented by formula (2) may be an E-isomer, Z-isomer, or a blend thereof, wherein the E-isomer and Z-isomer are geometric isomers that are present due to the partial structure >C=N-OH of the compound. The same applies to compounds other than the compound of formula (2) that include the wavy line.

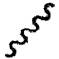

The compound represented by formula (2), a salt thereof, or a prodrug thereof is preferably a compound represented by the following formula (2A), a salt thereof, or a prodrug thereof. 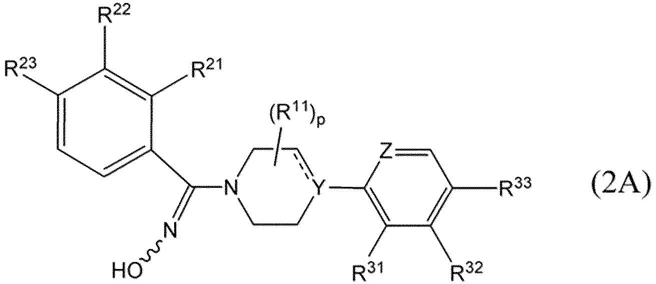 (wherein Z, Y, R¹¹, p, R²¹, R²², R²³, R³¹, R³², R³³, and ------ are as defined above.)

In the compound represented by formula (2A), Z, Y, R¹¹, p, R²¹, R²², R²³, R³¹, R³², R³³, and ------ are as defined above.

The compound represented by formula (2), a salt thereof, or a prodrug thereof is preferably a compound represented by the following formula (2B), a salt thereof, or a prodrug thereof. The compound represented by formula (2B) or a salt thereof is preferable as an intermediate compound in the production of the compound represented by formula (1).

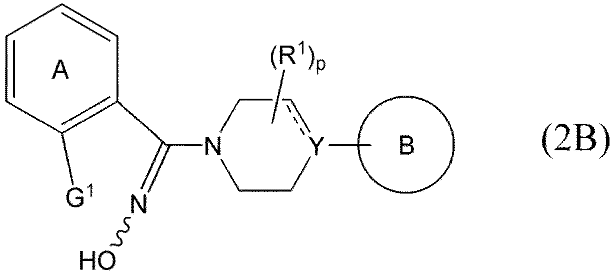

(wherein, A, B, Y, R¹, p, and ------ are as defined above, and G¹ is halogen, optionally halogen-substituted lower alkylsulfonyl, or benzenesulfonyl optionally substituted with lower alkyl or nitro).

In the compound represented by formula (2B), A, B, Y, R¹, p, and ------ are as defined above. The compound represented by formula (2B) or a salt thereof includes E-isomer and Z-isomer, which are geometric isomers that are present due to the partial structure >C=N-OH of the compound. When the compound represented by formula (2B) or a salt thereof is used as an intermediate compound in the production of the compound represented by formula (1), the E-isomer is preferable.

Examples of the halogen represented by G¹ include a chlorine atom, a fluorine atom, a bromine atom, and an iodine atom.

The lower alkylsulfonyl in the optionally halogen-substituted lower alkylsulfonyl represented by G¹ is as defined above. The lower alkylsulfonyl is a group in which lower alkyl is bound to sulfonyl. The lower alkyl may be substituted with halogen. Examples of the optionally halogen-substituted lower alkylsulfonyl include linear or branched C₁-C₆ alkyl (preferably C₁-C₄ alkyl, and more preferably C₁-C₃ alkyl) sulfonyl that may be substituted with one to three halogens. Specific examples include methanesulfonyl, ethanesulfonyl, trifluoromethanesulfonyl, and the like.

Examples of the benzenesulfonyl optionally substituted with lower alkyl represented by G¹ include benzenesulfonyl that may be substituted with one to three (preferably one or two, and more preferably one) linear or branched C₁-C₆ alkyl groups (preferably C₁-C₄ alkyl, and more preferably C₁-C₃ alkyl groups). Specific examples include p-toluenesulfonyl and the like.

Examples of the benzenesulfonyl optionally substituted with nitro represented by G¹ include benzenesulfonyl that may be substituted with one to three (preferably one) nitro groups. Specific examples include o-nitrobenzenesulfonyl, p-nitrobenzenesulfonyl, and the like.

G¹ is preferably a chlorine atom, a fluorine atom, a bromine atom, methanesulfonyl, ethanesulfonyl, trifluoromethanesulfonyl, p-toluenesulfonyl, or p-nitrobenzenesulfonyl. G¹ is more preferably a chlorine atom or a bromine atom.

The compound represented by formula (2), a salt thereof, or a prodrug thereof includes, for example, the following compounds, salts thereof, or prodrugs thereof.

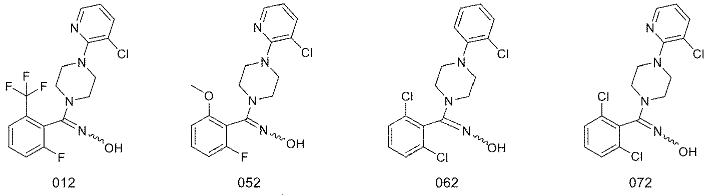

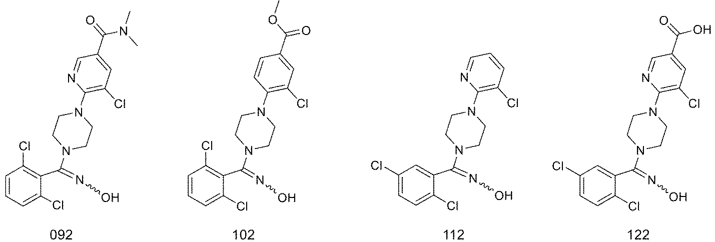

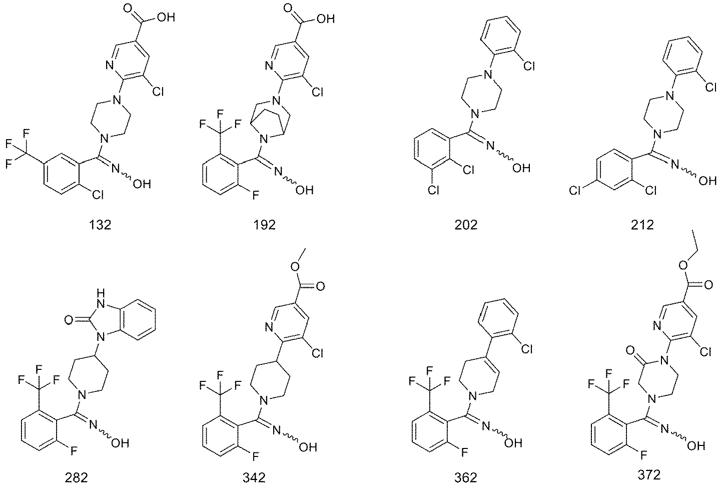

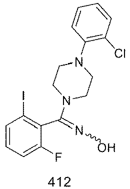

The compound represented by formula (2), a salt thereof, or a prodrug thereof is preferably Compound 062, Compound 202, Compound 362, or Compound 372; a salt thereof; or a prodrug thereof; and is more preferably (E)-isomer of Compound 202, (E)-isomer of Compound 362, (Z)-isomer of Compound 362, or (Z)-isomer of Compound 372; a salt thereof; or a prodrug thereof.

The compound described above can be produced, for example, by appropriate modification or a combination of any of the following: Production Methods 1 to 3 described below in detail, similar methods, known methods, and the like. The compounds to be used as starting material compounds may be used in the form of salts. The methods shown below are merely illustrative, and other methods can also be used as appropriate based on the knowledge of people skilled in organic synthesis. When 1,2-benzisothiazole or a derivative thereof or 1,2-benzisoxazole or a derivative thereof that is not a commercial product is used as a starting material compound, the compound can be produced and prepared with reference to the method disclosed in the following publication:
Advances in Heterocyclic Chemistry, Heterocyclic Chemistry in the 21st Century: A Tribute to Alan Katritzky, Elsevier, Cambridge (2017). R. A. Shastri, Review on Synthesis of 3-Substituted 1,2-Benzisoxazole Derivatives, Chem. Sci. Trans., 2016: 5; 8-20.

In each reaction for the production, functional groups can be protected as desired. With respect to the protecting groups and the technique of protection with protecting groups and deprotection, known methods, such as the method described in the following can be appropriately used: T. W. Greene and P. G. M. Wuts, Protective Groups in Organic Synthesis, 3rd Ed., John Wiley and Sons, Inc., New York (1999).

### Production Method 1

In an embodiment, the compound represented by formula (1) can be produced by the synthesis scheme illustrated in the following Reaction Formula-1. Specifically, the compound represented by formula (1) can be produced from the compound represented by formula (3) and the compound represented by formula (4).

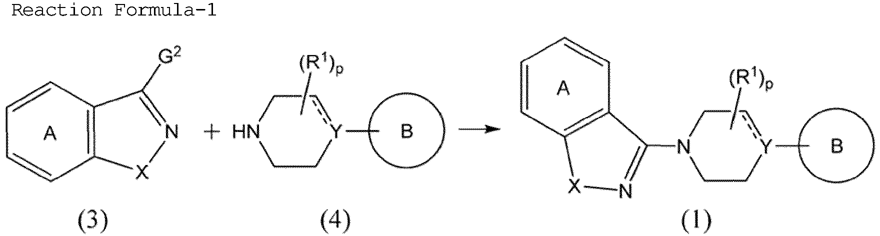

(wherein A, B, Y, R¹, p, and ------ are as defined above; and G² represents halogen, optionally halogen-substituted lower alkylsulfonyl, or benzenesulfonyl optionally substituted with lower alkyl or nitro.)

In Reaction Formula-1, A, B, Y, R¹, p, and ------ are as defined above.

Examples of halogen represented by G² include a chlorine atom, a fluorine atom, a bromine atom, and an iodine atom.

The "lower alkyl" in the optionally halogen-substituted lower alkylsulfonyl represented by G² is as defined above. The lower alkylsulfonyl is a group in which lower alkyl is bound to sulfonyl, and the lower alkyl may optionally be substituted with one or more halogens. Examples of the optionally halogen-substituted lower alkylsulfonyl include linear or branched C₁-C₆ alkyl (preferably C₁-C₄ alkyl, more preferably C₁-C₃ alkyl) sulfonyl that may be substituted with 1 to 3 halogens, and specifically, methanesulfonyl, ethanesulfonyl, trifluoromethanesulfonyl, and the like.

Examples of the benzenesulfonyl optionally substituted with lower alkyl represented by G² include benzenesulfonyl that may be substituted with 1 to 3 linear or branched C₁-C₆ alkyl groups (preferably C₁-C₄ alkyl, more preferably C₁-C₃ alkyl), and specifically, p-toluenesulfonyl and the like.

Examples of the benzenesulfonyl optionally substituted with nitro represented by G² include benzenesulfonyl that may be substituted with 1 to 3 (preferably 1) nitro groups, specifically, o-nitrobenzenesulfonyl, p-nitrobenzenesulfonyl, and the like.

G² is preferably a chlorine atom, a fluorine atom, a bromine atom, methanesulfonyl, ethanesulfonyl, trifluoromethanesulfonyl, p-toluenesulfonyl, or p-nitrobenzenesulfonyl.

The reaction of the compound represented by formula (3) with the compound represented by formula (4) can be performed, for example, in an inert solvent in the presence or absence of a base. An activating reagent may optionally be further added to the reaction system. The compound represented by formula (3) and the compound represented by formula (4) are known compounds and can be produced by a known method.

Examples of inert solvents include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the base for use is typically 1 mol or more, preferably 1 to 5 mol, and more preferably 1 to 2 mol, per mol of the compound represented by formula (4).

The amount of the compound represented by formula (3) for use is typically 0.2 mol or more, preferably 0.2 to 2 mol, and more preferably 0.2 to 1.5 mol, per mol of the compound represented by formula (4).

The reaction temperature is typically -50°C to 180°C, preferably -30°C to 180°C, and more preferably -10°C to 180°C. Microwaves may be used for facilitating the reaction, and the reaction temperature in this case is, for example, 80°C to 180°C, and preferably 100°C to 180°C. The reaction time is typically 10 minutes to 48 hours, and preferably 10 minutes to 24 hours.

### Production Method 2

In an embodiment, the compound represented by formula (1B) can be produced by the synthesis scheme illustrated in the following Reaction Formula-2. Specifically, the compound represented by formula (1B) can be produced from the compound represented by formula (5) and the compound represented by formula (6).

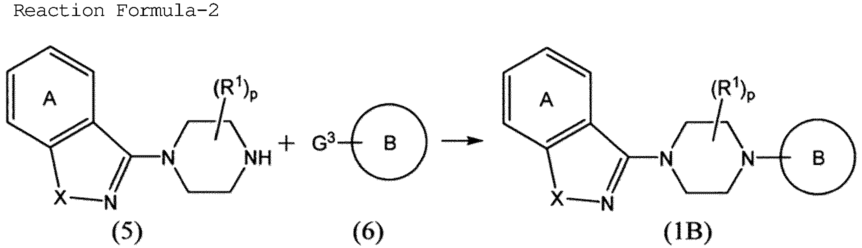

(wherein A, B, X, R¹, and p are as defined above, and G³ represents halogen, optionally halogen-substituted lower alkylsulfonyl, or benzenesulfonyl optionally substituted with lower alkyl or nitro.)

In Reaction Formula-2, A, B, X, R¹, and p are as defined above.

Examples of halogens represented by G³ include a chlorine atom, a fluorine atom, a bromine atom, and an iodine atom.

The "lower alkyl" in the optionally halogen-substituted lower alkylsulfonyl represented by G³ is as defined above. The lower alkylsulfonyl is a group in which lower alkyl is bound to sulfonyl, and the lower alkyl may optionally be substituted with one or more halogens. Examples of the optionally halogen-substituted lower alkylsulfonyl include linear or branched C₁-C₆ alkyl (preferably C₁-C₄ alkyl, more preferably C₁-C₃ alkyl) sulfonyl that may be substituted with 1 to 3 halogens, specifically, methanesulfonyl, ethanesulfonyl, trifluoromethanesulfonyl, and the like.

Examples of the benzenesulfonyl optionally substituted with lower alkyl represented by G³ include benzenesulfonyl that may be substituted with 1 to 3 linear or branched C₁-C₆ alkyl groups (preferably C₁-C₄ alkyl, more preferably C₁-C₃ alkyl), and specifically, p-toluenesulfonyl, and the like.

Examples of the benzenesulfonyl optionally substituted with nitro represented by G³ include benzenesulfonyl that may be substituted with 1 to 3 (preferably 1) nitro groups, specifically, o-nitrobenzenesulfonyl, p-nitrobenzenesulfonyl, and the like.

G³ is preferably a chlorine atom, a fluorine atom, a bromine atom, methanesulfonyl, ethanesulfonyl, trifluoromethanesulfonyl, p-toluenesulfonyl, or p-nitrobenzenesulfonyl.

The compound represented by formula (1B) can be obtained by coupling the compound represented by formula (5) with the compound represented by formula (6). The compound represented by formula (5) and the compound represented by formula (6) are known compounds and can be produced by a known method.

This reaction can be performed, for example, in an inert solvent in the presence of a base.

Examples of inert solvents include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the compound represented by formula (6) for use is typically 0.5 mol or more, further 1 mol or more, preferably 0.9 to 2 mol, and more preferably 0.9 to 1.5 mol, per mol of the compound represented by formula (5).

The amount of the base for use is typically 1 mol or more, preferably 1 to 5 mol, and more preferably 1 to 2 mol, per mol of the compound represented by formula (5).

The reaction temperature is typically 30°C to a temperature higher than the boiling point of the solvent by 10°C, and preferably 80°C to a temperature higher than the boiling point of the solvent by 10°C. To facilitate the reaction, microwaves may be used. The reaction temperature in this case is, for example, 80°C to 180°C, and preferably 100°C to 180°C. The reaction time is typically 10 minutes to 48 hours, and preferably 10 minutes to 24 hours.

Additionally, the reaction of the compound represented by formula (5) with the compound represented by formula (6) can be performed by using the Buchwald reaction. For example, in the presence of a palladium catalyst, a phosphine ligand, and a base, the compound represented by formula (5) is reacted with the compound represented by formula (6) in a solvent.

Examples of palladium catalysts include divalent palladium catalysts, such as Pd(OAc)₂, PdCl₂, allyl palladium(II) chloride (dimer), bis(acetonitrile)palladium(II) dichloride, and bis(benzonitrile)palladium(II) dichloride; and zerovalent palladium catalysts, such as Pd₂(dba)₃ (tris(dibenzylideneacetone) dipalladium(0)), bis(dibenzylideneacetone) palladium(0), and palladium on carbon (Pd/C).

Examples of phosphine ligands include bidentate phosphine ligands, such as BINAP (2,2'-bis(diphenylphosphanyl)-1,1'-bisnaphthalene) and Xphos (2-dicyclohexylphosphino-2',4',6'-triisopropylbiphenyl).

Examples of bases include strong bases, such as t-BuONa (sodium t-butoxide).

The amount of the compound represented by formula (6) for use in this reaction is typically 0.5 mol or more, further 1 mol or more, preferably 0.9 to 2 mol, and more preferably 1 to 1.5 mol, per mol of the compound represented by formula (5).

The amount of the palladium catalyst for use is typically 0.005 to 1 mol, and preferably 0.01 to 0.2 mol, per mol of the compound represented by formula (5).

The amount of the phosphine ligand for use is typically 0.5 to 5 mol, and preferably 1 to 2 mol, per mol of the palladium catalyst.

The amount of the base for use is typically 0.5 mol or more, further 1 mol or more, and preferably 1 to 2 mol, per mol of the compound represented by formula (5).

The reaction temperature is typically 40°C to 150°C, and preferably 80°C to 110°C. The reaction time is typically 1 to 24 hours, and preferably 3 to 12 hours.

### Production Method 3

In an embodiment, the compound represented by formula (1) or (2) can be produced by the synthesis scheme illustrated in the following Reaction Formula-3. Specifically, the compound represented by formula (1C) can be produced by converting the compound represented by formula (7) into the compound represented by formula (8), reacting the compound represented by formula (8) with the compound represented by formula (4) to produce the oxime compound represented by formula (2B), and ring-closing the oxime compound represented by formula (2B). A person skilled in the art would be able to understand that the compound represented by formula (2) can be produced by using an appropriate, corresponding compound that has an optionally substituted benzene ring A, instead of the compound represented by formula (7) or (8), in the reaction illustrated in Reaction Formula-3. The compound represented by formula (7) is a known compound and can be produced by a known method.

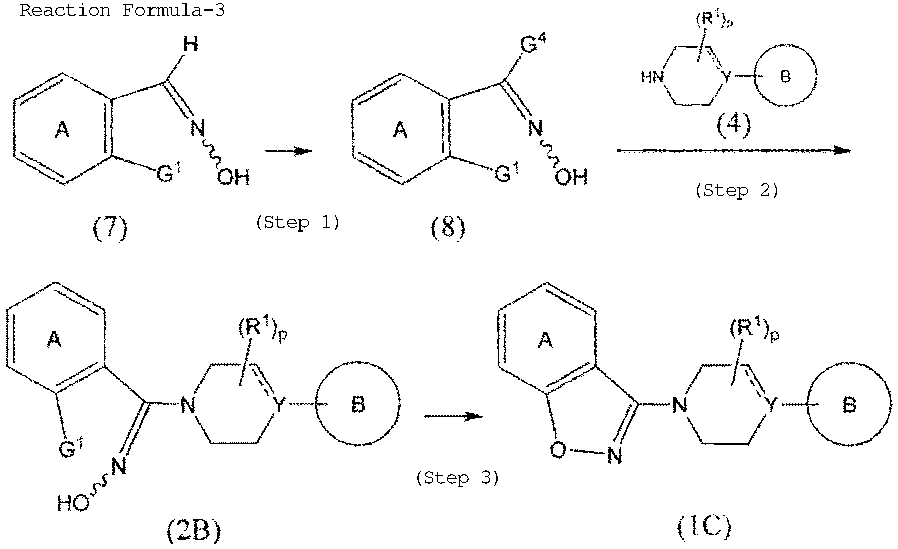

(wherein A, B, Y, R¹, p, G¹, and - - - - - - are as defined above; and G⁴ represents halogen.)

In Reaction Formula-3, A, B, Y, R¹, p, G¹, and - - - - - - are as defined above.

Examples of halogens represented by G⁴ include a chlorine atom, a fluorine atom, a bromine atom, and an iodine atom.

Step 1 (i.e., the step of converting the compound represented by formula (7) into the compound represented by formula (8)) can be performed, for example, by reacting the compound represented by formula (7) with a halogenating agent in an inert solvent.

Examples of inert solvents for use in this reaction include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of halogenating agents include typical halogenating agents, such as N-bromosuccinimide and N-chlorosuccinimide.

The amount of the halogenating agent for use is typically an equimolar amount to an excess molar amount, preferably 1- to 5-fold mol, and more preferably 1- to 2-fold mol, based on the compound represented by formula (7).

The reaction temperature is typically -30 to 150°C, preferably -10 to 100°C, and more preferably -10 to 40°C. The reaction time is typically 10 minutes to 48 hours, preferably 10 minutes to 24 hours, and more preferably 30 minutes to 18 hours.

Step 2 (i.e., the step of reacting the compound represented by formula (8) with the compound represented by formula (4) to synthesize the compound represented by formula (2B)) can be performed, for example, in an inert solvent in the presence of a base.

Examples of inert solvents for use in this reaction include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the compound represented by formula (8) for use is typically 0.5 mol or more, 0.8 mol or more, preferably 0.9 to 2 mol, and more preferably 0.9 to 1.5 mol, per mol of the compound represented by formula (4).

The amount of the base for use is typically 1 mol or more, preferably 1- to 5-fold mol, and more preferably 1- to 2-fold mol, per mol of the compound represented by formula (4).

The reaction temperature is typically -20°C to a temperature higher than the boiling point of the solvent by 10°C, and preferably 0°C to 40°C. The reaction time is typically 10 minutes to 48 hours, preferably 10 minutes to 24 hours, and more preferably 30 minutes to 18 hours.

Step 3 (i.e., the step of ring-closing the compound represented by formula (2B) to convert the compound represented by formula (2B) into the compound represented by formula (1)) can be performed, for example, in an inert solvent in the presence of a base.

The compound represented by formula (2B) exists in both forms of (E)-isomer and (Z)-isomer, which are geometric isomers. From the standpoint of less heating required in the ring-closing reaction, (E)-isomer is preferable.

In the reaction of step 2, typically, the (Z)-isomer is obtained as the main product. By subjecting the (Z)-isomer to acidic conditions, a preferable (E)-isomer form can be obtained by isomerization. For example, the (E)-isomer can be obtained almost quantitatively by treating a mixture of the (E)-isomer and (Z)-isomer with a catalytic amount of an acid in an inert solvent. Therefore, an isomerization reaction may be performed after step 2 and before step 3.

Examples of inert solvents for the isomerization reaction include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of acids for the isomerization reaction include inorganic acids, such as sulfuric acid, hydrochloric acid, bromic acid, and perchloric acid; carboxylic acids, such as acetic acid, lactic acid, oxalic acid, and trifluoroacetic acid; sulfonic acids, such as methanesulfonic acid, benzenesulfonic acid, p-toluenesulfonic acid, camphorsulfonic acid, and trifluoromethanesulfonic acid; and Lewis acids, such as aluminum chloride, boron trifluoride, titanium tetrachloride, copper acetate, copper chloride, iron chloride, zinc chloride, and tin chloride.

The amount of the acid for use may be a catalytic amount and is for example, 0.001- to 0.3-fold mol, and preferably 0.01- to 0.2-fold mol, per mol of the compound represented by formula (2B).

The reaction temperature in the isomerization reaction is typically room temperature to the boiling point of the solvent, and preferably 40°C to the boiling point of the solvent. The reaction time is typically 10 minutes to 24 hours, and preferably 1 to 6 hours.

Examples of inert solvents for step 3 include ether solvents, such as diethyl ether, tetrahydrofuran (THF), dioxane, and dimethoxymethane; aromatic hydrocarbon solvents, such as toluene, benzene, and xylene; halogenated hydrocarbon solvents, such as dichloromethane, chloroform, dichloroethane, and carbon tetrachloride; ketone solvents, such as acetone; aprotic solvents, such as dimethylsulfoxide, N,N-dimethylformamide (DMF), and acetonitrile; and pyridine. These solvents may also be used in a combination of two or more in appropriate proportions.

Examples of bases include metal hydrides, such as sodium hydride and potassium hydride; metal hydroxides, such as potassium hydroxide and sodium hydroxide; metal carbonates, such as potassium carbonate, potassium hydrogen carbonate, sodium carbonate, sodium hydrogen carbonate, and cesium carbonate; alkyl amines, such as triethylamine and ethyldiisopropylamine; and metal alkoxides, such as sodium methoxide and potassium t-butoxide.

The amount of the base for use is typically 1 mol or more, preferably 1- to 5-fold mol, and more preferably 1- to 2-fold mol, per mol of the compound represented by formula (2B).

The reaction temperature is typically 50°C to a temperature higher than the boiling point of the solvent by 10°C, and preferably 80°C to a temperature higher than the boiling point of the solvent by 10°C. To facilitate the reaction, microwaves may be used. In this case, the reaction temperature is, for example 80°C to 180°C, and preferably 100°C to 180°C. The reaction time is typically 10 minutes to 8 hours, and preferably 10 minutes to 2 hours.

The compound represented by formula (1) or (2) according to the present invention, intermediate compounds thereof, and starting material compounds thereof can be produced by the production methods described above, and can also be produced with reference to an already-known or publicly known technique at the time the present application was filed (e.g., B.R. Kiran et al., SYNTHESIS, EVALUATION OF ANALGESIC AND ANTIINFLAMMATORY ACTIVITIES OF SUBSTITUTED 1,2-BENZOXAZOLONE AND 3-CHLORO-1,2-BENZOXAZOLE DERIVATIVES, International Journal of Pharmaceutical Sciences and Research, 2015; 6: 2918-2925) in accordance with the synthesis methods disclosed in the Examples of the present specification.

The functional groups of the starting material compounds and intermediate compounds illustrated in the reaction schemes above may optionally be protected by an appropriate protective group, using a known method, before the compounds are subjected to a reaction, and be deprotected using a known method after completion of the reaction.

The target compounds obtained in accordance with the reaction schemes above can be isolated and purified. For example, after the reaction mixture is cooled, the crude reaction product is subjected to isolation procedures, such as filtration, concentration, and extraction, in order to separate the crude reaction product; the crude reaction product is then subjected to typical purification procedures, such as column chromatography and recrystallization, thereby isolating and purifying a target compound from the reaction mixture.

The starting material compounds and the compound represented by formula (1) or (2) illustrated in the reaction schemes above include compounds in the form of solvate in which a solvent is added (e.g., a hydrate and solvate of ethanol).

When the compound represented by formula (1) or (2), the intermediate compounds obtained in the reaction schemes, or the starting material compounds have isomers, the compound represented by formula (1) or (2), the intermediate compounds obtained in the reaction schemes, or the starting material compounds include all of these isomers. The isomers include an isomer due to a double bond, a ring, or a fused ring (an E-, Z-, sis-, or trans-form); an isomer, for example, due to the presence of an asymmetric carbon (an R- or S-form, α- or β-form, enantiomer, or diastereomer); an optically active form that exhibits optical rotations (a D-, L-, d-, or l-form); a polar form due to chromatographic separation (a high polar form and low polar form); an equilibrium compound; a rotamer; a mixture thereof of any proportions; and a racemic mixture (isomers such as a geometric isomer, a stereoisomer, and an optical isomer). For example, optical isomers can be separated by using various known resolution methods (e.g., optical resolution by crystallization and direct optical resolution by chromatography).

The salt of the compound represented by formula (1) or (2) includes all pharmaceutically acceptable salts. The pharmaceutically acceptable salt can be any pharmaceutically acceptable salt. Examples include alkali metal salts, such as sodium salts and potassium salts; alkaline-earth metal salts, such as calcium salts and magnesium salts; inorganic metal salts, such as zinc salts; organic base salts, such as triethylamine, triethanolamine, trihydroxymethyl amino methane, and amino acid; inorganic acid salts, such as hydrochloride, hydrobromate, sulfate, phosphate, and nitrate; and organic acid salts, such as acetate, carbonate, propionate, succinate, lactate, malate, tartrate, citrate, maleate, fumarate, methanesulfonate, p-toluenesulfonate, benzenesulfonate, and ascorbate. These salts can be produced in accordance with an ordinary method.

The variety of isomers can be isolated by a known separation method. For example, racemates can be led to sterically pure isomers by a typical optical resolution method (e.g., optical resolution by crystallization and direct optical resolution by chromatography). An optically active compound can be produced by using a suitable, optically active starting material.

The starting material compounds, intermediate compounds, and target compounds described in the reaction schemes above can be used in their suitable salt form.

In the present invention, in the compound represented by formula (1) or (2), a salt thereof, or a prodrug thereof, one or multiple atoms may be replaced by one or multiple isotopes. Examples of isotopes include deuterium (2H), tritium (3H), 13C, 14N, 180, and the like.

The pharmaceutical composition according to the present invention may be a preparation of the compound represented by formula (1) or (2), a salt thereof, or a prodrug thereof in a typical pharmaceutical composition form, and may be prepared by using the compound, a salt thereof, or a prodrug thereof and a pharmaceutically acceptable carrier. Examples of carriers include diluents and excipients, such as typically used fillers, extenders, binders, moisturizing agents, disintegrators, surfactants, and lubricants.

In the present invention, the prodrug refers to a compound that is converted into the compound represented by formula (1) or (2) through a reaction in vivo (e.g., an enzyme reaction and a reaction caused by stomach acid). For example, when the compound represented by formula (1) or (2) has carboxyl, the prodrug is a compound in which the carboxyl is converted into an ester. Examples of the esters include methyl ester, ethyl ester, 1-propyl ester, 2-propyl ester, pivaloyloxy methyl ester, acetyloxymethyl ester, cyclohexyl acetyloxymethyl ester, 1-methyl cyclohexyl carbonyloxy methyl ester, ethyloxy carbonyloxy-1-ethyl ester, cyclohexyloxy carbonyloxy-1-ethyl ester, and the like.

The pharmaceutical composition according to the present invention can be selected from various dosage forms according to the purpose of treatment. Typical examples include tablets, pills, powders, liquids, suspensions, emulsions, granules, capsules, suppositories, injectable drugs (e.g., liquids and suspensions), ointments, inhalants, ear drops, and the like. Among these, the pharmaceutical composition according to the present invention is preferably a preparation for oral administration, a preparation for transdermal administration, a preparation for subcutaneous administration, a preparation for topical administration, or an injectable drug, and more preferably a preparation for oral administration.

The carrier for use in forming tablets can be selected from a wide range of known carriers. Examples include excipients, such as lactose, sucrose, sodium chloride, glucose, urea, starch, calcium carbonate, kaolin, and crystalline cellulose; binders, such as water, ethanol, propanol, simple syrup, a dextrose solution, a starch solution, a gelatin solution, methylcellulose, potassium phosphate, polyvinyl pyrrolidone, carboxy methylcellulose, and shellac; disintegrators, such as sodium alginate, dry starch, agar powder, laminaran powder, calcium carbonate, sodium hydrogen carbonate, polyoxyethylene sorbitan fatty acid esters, sodium lauryl sulfate, stearic acid monoglyceride, starch, and lactose; sorbefacients, such as a quaternary ammonium base and sodium lauryl sulfate; disintegration inhibitors, such as stearin, cocoa butter, and hydrogenated oil; moisturizers, such as glycerol and starch; adsorbents, such as starch, lactose, kaolin, bentonite, and colloidal silica; lubricants, such as purified talc, stearate, boric acid powder, and polyethylene glycol; and the like.

Tablets may further optionally be formed into tablets with typical coating, such as sugarcoated tablets, gelatin-coated tablets, enteric coating tablets, film coating tablets, double-layered tablets, or multi-layered tablets.

Carriers for use in preparing pills can be selected from a wide range of known carriers. Examples include excipients, such as glucose, lactose, starch, cacao oil, hydrogenated vegetable oil, kaolin, and talc; binders, such as powdered gum arabic, powdered tragacanth, gelatin, and ethanol; and disintegrators, such as laminaran and agar.

Carriers for use in preparing suppositories can be selected from a wide range of known carriers. Examples include polyethylene glycol, cacao oil, higher alcohols, esters of higher alcohols, gelatin, semisynthetic glycerides, and the like.

When the pharmaceutical composition according to the present invention is prepared into an injectable drug, it is preferred that the liquid agent, emulsion, or suspension be sterilized and be isotonic with blood. Diluents for use in preparing the liquid agent, emulsion, and suspension can be selected from a wide range of known diluents. Examples include water, ethanol, propylene glycol, polyoxylated isostearyl alcohols, ethoxylated isostearyl alcohols, polyoxyethylene sorbitan fatty acid esters, and the like. In the case of an injectable drug, the medical drug preparation can contain sodium chloride, glycerol, glucose, etc. in an amount sufficient to form an isotonic solution; and further can contain a typical solubilizing agent, a buffer, a soothing agent, etc., with further optionally a colorant, a preservative, an aroma component, a flavoring, a sweetener, and other medical drugs.

Ointments have forms such as a paste, cream, and gel. Examples of diluents for use in preparing an ointment of any of these forms include white petrolatum, paraffin, glycerol, cellulose derivatives, polyethylene glycol, silicone, and bentonite.

Inhalants are preparations intended to be applied to the bronchus or lungs by making a subject inhale an active ingredient in the form of aerosol. Inhalants include powdered inhalants, inhalant liquid agents, inhalant aerosol agents, and the like. Powdered inhalants refer to the preparations by which powdered solid particles in the form of aerosol are inhaled. Powdered inhalants can be typically produced by mixing an active ingredient in the form of fine particles with optional additives, such as lactose, to form a homogeneous mixture. Inhalant liquid agents refer to liquid inhalants that are applied with a device, such as a nebulizer. Inhalant liquid agents are typically produced by adding a solvent, a suitable isotonic agent, a pH adjuster, etc. to an active ingredient, and mixing them. Inhalant aerosol agents refer to metered-dose spray inhalants that spray a specific amount of an active ingredient together with a propellant that fills a container. Inhalant aerosol agents can be typically produced by adding a solvent, a suitable dispersant, stabilizer, etc. to an active ingredient to form a solution or suspension, filling a pressure-resistant container with the solution or suspension together with a liquid propellant, and attaching a metering valve to the container.

The pharmaceutical composition according to the present invention may optionally contain a colorant, a preservative, an aroma component, a flavoring, a sweetener, and other medical drugs.

The amount of the compound represented by formula (1) or (2), a salt thereof, or a prodrug thereof contained in the pharmaceutical composition according to the present invention can be any amount and can be suitably selected from a wide range of amounts. The compound according to the present invention, a salt thereof, or a prodrug thereof is present in an amount of typically 0.5 to 90 wt%, 1 to 85 wt%, and preferably 1 to 80 wt% in the pharmaceutical composition.

The method for administering the pharmaceutical composition according to the present invention is not particularly limited, and can be administered by a method according to the dosage form, patient's age, gender, disease condition, and other conditions. For example, the pharmaceutical composition according to the present invention in the form of a tablet, pill, liquid agent, suspension, emulsion, granule, or capsule can be orally administered. The pharmaceutical composition according to the present invention in the form of an injectable drug alone or in the form of an injectable drug mixed with a typical replacement fluid, such as glucose and amino acids, can be intravenously administered; or the pharmaceutical composition in the form of an injectable drug alone can be optionally administered, for example, intramuscularly, intradermally, subcutaneously, or intraperitoneally. The pharmaceutical composition according to the present invention in the form of a suppository is intrarectally administered. The pharmaceutical composition according to the present invention in the form of an inhalant is administered nasally. The pharmaceutical composition according to the present invention in the form of an ear drop is administered intra-aurally. The administration method is preferably oral administration or injection administration (including subcutaneous administration, intramuscular administration, intravenous administration, and intrathecal administration), more preferably oral administration or subcutaneous administration, and even more preferably oral administration.

The dose of the pharmaceutical composition according to the present invention can be determined taking into consideration the regimen, patient's age, gender, severity of disease, and other conditions. The pharmaceutical composition is administered such that the amount of the compound represented by formula (1) or (2), a salt thereof, or a prodrug thereof as an active ingredient is, for example, 0.01 to 100 mg, 0.05 to 100 mg, or 0.1 to 100 mg, and preferably 0.1 to 50 mg, per kilogram of body weight daily in one time or in several times per day, or every two days, three days, four days, five days, six days, one week, two weeks, or four weeks. Because the dose varies depending on various conditions, a dose lower than a dose within the ranges above may be sufficient in some cases, and a dose higher than a dose within the ranges above may be necessary in other cases.

The pharmaceutical composition according to the present invention may be combined with one or more other medicinal agents to form a combination drug. Examples of other medicinal agents include medicinal agents having an action of attenuating a kidney disease, an action of suppressing deterioration of kidney function, or an action of ameliorating kidney function. The medicinal agents having an action of attenuating a kidney disease, an action of suppressing deterioration of kidney function, or an action of ameliorating kidney function for use may be known medicinal agents. Examples of the medicinal agents having an action of attenuating a kidney disease, an action of suppressing deterioration of kidney function, or an action of ameliorating kidney function for use in combination include SGLT2 inhibitors, such as dapagliflozin, empagliflozin, and canagliflozin; angiotensin II receptor antagonists, such as candesartan and valsartan; angiotensin-converting enzyme inhibitors, such as enalapril, imidapril, and perindopril; mineralocorticoid receptor antagonists, such as spironolactone and eplerenone; calcium antagonists, such as amlodipine and nifedipine; loop diuretics, such as furosemide, azosemide, and torasemide; corticosteroids, such as prednisolone; immunosuppressants (calcineurin inhibitors), such as ciclosporin; and the like.

The present invention may include a method for preventing and/or treating a kidney disease, comprising administering an effective amount of the compound represented by formula (1) or (2), a pharmaceutically acceptable salt thereof, or a prodrug thereof to a patient in need of preventing and/or treating a kidney disease. The present invention may include a method for attenuating the progression of a kidney disease, comprising administering an effective amount of the compound represented by formula (1) or (2), a pharmaceutically acceptable salt thereof, or a prodrug thereof to a patient in need of attenuating the progression of a kidney disease. Furthermore, the present invention may include a method for suppressing deterioration of and/or ameliorating kidney function, comprising administering an effective amount of the compound represented by formula (1) or (2), a pharmaceutically acceptable salt thereof, or a prodrug thereof to a patient in need of suppressing deterioration of and/or ameliorating kidney function.

The subject of the administration is preferably a subject with a high urinary protein level.

A "high urinary protein level" means that the protein concentration in the urine is, for example, 30 mg/dL or more, preferably 100 mg/dL or more, more preferably 300 mg/dL or more, and even more preferably 1000 mg/dL or more. The upper limit is not particularly limited and is typically 10000 mg/dL or less.

The compound represented by formula (1) or (2), a salt thereof, or a prodrug thereof has an action of preventing and/or treating a kidney disease (in other words, an action of attenuating the progression of a kidney disease). The compound represented by formula (1) or (2), a salt thereof, or a prodrug thereof has an action of suppressing deterioration of and/or ameliorating kidney function. The action of suppressing deterioration of and/or ameliorating kidney function refers to an action of suppressing an increase in the protein levels in the urine, an action of reducing the protein levels in the urine, and the like. The action of reducing the protein levels in the urine may be, for example, an action of reducing the protein levels in the urine by 20 mg/dL or more, 30 mg/dL or more, 50 mg/dL or more, 80 mg/dL or more, or the like, preferably 100 mg/dL or more, more preferably 120 mg/dL or more, and even more preferably 150 mg/dL or more. In the action of reducing the protein levels in the urine, the upper limit of the reduction amount of the protein levels is not particularly limited and may be, for example, 10000 mg/dL or less, 5000 mg/dL or less, 2000 mg/dL or less, 1000 mg/dL or less, or the like.

Kidney diseases can be broadly classified into glomerular diseases, vascular diseases, tubular or interstitial diseases, and the like, depending on the site of the injury.

Examples of glomerular diseases include primary glomerular diseases, such as chronic glomerulonephritis (IgA nephropathy, membranous nephropathy, minimal change nephrotic syndrome, focal segmental glomerulosclerosis (FSGS), crescentic glomerulonephritis, and membranoproliferative glomerulonephritis); secondary glomerular diseases, such as diabetic nephropathy, lupus nephritis, microscopic polyangiitis (ANCA-related vasculitis), and hepatitis virus-associated nephropathy; and hereditary or congenital glomerular diseases, such as benign familial hematuria, Alport syndrome, and Fabry disease.

Examples of vascular diseases include hypertensive nephropathy (nephrosclerosis), renal artery stenosis (fibromuscular dysplasia, aortitis syndrome, arteriosclerosis), cholesterol embolism, renal vein thrombosis, ischemic nephropathy, and the like.

Examples of tubular or interstitial diseases include chronic interstitial nephritis, gouty nephropathy, drug-induced renal dysfunction, polycystic kidney disease, nephronophthisis, and the like. A tubular or interstitial disease, excluding drug-induced renal dysfunction, is preferred.

The kidney disease is preferably a glomerular disease. The glomerular disease is preferably chronic glomerulonephritis and diabetic nephropathy,
more preferably IgA nephropathy, membranous nephropathy, minimal change nephrotic syndrome, focal segmental glomerulosclerosis, crescentic glomerulonephritis, membranoproliferative glomerulonephritis, and diabetic nephropathy,
even more preferably IgA nephropathy, membranous nephropathy, minimal change nephrotic syndrome, focal segmental glomerulosclerosis, crescentic glomerulonephritis, and membranoproliferative glomerulonephritis,
and particularly preferably focal segmental glomerulosclerosis and minimal change nephrotic syndrome.

Of the diseases listed above as examples, the kidney disease is preferably a disease accompanied by proteinuria. The kidney disease accompanied by proteinuria may be, for example, minimal change nephrotic syndrome, focal segmental glomerulosclerosis, diabetic nephropathy, IgA nephropathy, or membranous nephropathy, and is preferably minimal change nephrotic syndrome, focal segmental glomerulosclerosis, or diabetic nephropathy, and more preferably minimal change nephrotic syndrome or focal segmental glomerulosclerosis.

The kidney disease may be a kidney disease excluding at least one, at least two, at least three, at least four, at least five, or all of the following diseases.
Kidney diseases that can be excluded:
- Acute kidney injury (in particular, kidney injury caused by administration of a platinum-containing drug)
- Kidney aging
- Kidney function deterioration due to aging
- Diabetic kidney disease
- Obesity-related kidney disease (e.g. obesity-related tubulopathy)
- Kidney fibrosis

The kidney disease may be a kidney disease excluding acute kidney injury, in particular, a kidney disease excluding kidney injury caused by administration of a platinum-containing drug.

The kidney disease may be a kidney disease excluding kidney aging, kidney function deterioration due to aging, diabetic kidney disease, and obesity-related kidney disease (e.g., obesity-related tubulopathy).

The kidney disease may be a kidney disease excluding acute kidney injury, kidney aging, kidney function deterioration due to aging, diabetic kidney disease, and obesity-related kidney disease (e.g., obesity-related tubulopathy).

The kidney disease may be a kidney disease excluding kidney fibrosis, acute kidney injury, kidney aging, kidney function deterioration due to aging, diabetic kidney disease, and obesity-related kidney disease (e.g., obesity-related tubulopathy).

The compound represented by formula (1) or (2) is not easily metabolized in the liver. Furthermore, the compound is highly soluble in PBS and is thus advantageous in forming it into a preparation; additionally, due to its high membrane permeability, the compound is advantageous in bioavailability. Thus, the compound represented by formula (1) or (2) is excellent in pharmacokinetics.

### Examples

The present invention is described below in more detail with reference to Examples and the like. However, the present invention is not limited to these Examples.

### Production Example 1

In accordance with the methods described in Examples 1 to 43 of WO2021/079962, the following compounds were produced.
Compounds represented by formula (1):
   Compound 011, Compound 021, Compound 031, Compound 041, Compound 051, Compound 061, Compound 071, Compound 081, Compound 091, Compound 101, Compound 111, Compound 121, Compound 131, Compound 141, Compound 151, Compound 161, Compound 171, Compound 181, Compound 191, Compound 201, Compound 211, Compound 221, Compound 231, Compound 241, Compound 251, Compound 261, Compound 271, Compound 281, Compound 291, Compound 301, Compound 311, Compound 321, Compound 331, Compound 341, Compound 351, Compound 361, Compound 371, Compound 381, Compound 391, Compound 401, Compound 411, Compound 421, and Compound 431; and
compounds represented by formula (2):
   Compound 012, Compound 052, Compound 062, Compound 072, Compound 082, Compound 092, Compound 102, Compound 112, Compound 132, Compound 192, Compound 202, Compound 212, Compound 282, Compound 342, Compound 362, Compound 372, and Compound 412.
   5-Chloro-6-(4-(4-(trifluoromethyl)benzo[d]isoxazol-3-yl)piperazin-1-yl)nicotinic acid (Compound 011)
   (E)-5-Chloro-6-(4-((2-fluoro-6-(trifluoromethyl)phenyl)(hydroxyimino)methyl)piperazin-1-yl)nicotinic acid (Compound 012(E)) and its Z-isomer (Compound 012(Z))
   5-Chloro-N-methyl-6-(4-(4-(trifluoromethyl)benzo[d]isoxazol-3-yl)piperazin-1-yl)nicotinamide (Compound 021)
   (S)-5-Chloro-6-(3-methyl-4-(4-(trifluoromethyl)benzo[d]isoxazol-3-yl)piperazin-1-yl)nicotinic acid (Compound 031)
   (R)-5-Chloro-6-(3-methyl-4-(4-(trifluoromethyl)benzo[d]isoxazol-3-yl)piperazin-1-yl)nicotinic acid (Compound 041)
   5-Chloro-6-(4-(4-(methoxy)benzo[d]isoxazol-3-yl)piperazin-1-yl)nicotinic acid (Compound 051)
   5-Chloro-6-(4-((2-fluoro-6-methoxyphenyl(hydroxyimino)methyl)piperazin-1-yl)nicotinic acid (Compound 052)
   4-Chloro-3-(4-(2-chlorophenyl)piperazin-1-yl)benzo[d]isoxazole (Compound 061)
   (E)-(4-(2-Chlorophenyl)piperazin-1-yl)(2,6-dichlorophenyl)methanone oxime (Compound 062(E)) and its Z-isomer (Compound 062(Z))
   4-Chloro-3-(4-(3-chloropyridin-2-yl)piperazin-1-yl)benzo[d]isoxazole (Compound 071)
   (E)-(4-(3-Chloropyridin-2-yl)piperazin-1-yl) (2,6-dichlorophenyl)methanone oxime (Compound 072(E)) and its Z-isomer (Compound 072(Z))
   5-Chloro-6-(4-(4-chlorobenzo[d]isoxazol-3-yl)piperazin-1-yl)nicotinic acid (Compound 081)
   5-Chloro-6-(4-((2,6-dichlorophenyl)(hydroxyimino)methyl)piperazin-1-yl)nicotinic acid (Compound 082)
   5-Chloro-6-(4-(4-chlorobenzo[d]isoxazol-3-yl)piperazin-1-yl)N,N-dimethylnicotinamide (Compound 091)
   (E)-5-Chloro-6-(4-((2,6-dichlorophenyl)(hydroxyimino)methyl)piperazin-1-yl)-N,N-dimethylnicotinamide (Compound 092(E)) and its Z-isomer (Compound 092(Z))
   3-Chloro-4-(4-(4-chlorobenzo[d]isoxazol-3-yl)piperazin-1-yl)benzoic acid (Compound 101)
   3-Chloro-4-(4-((2,6-dichlorophenyl)(hydroxyimino)methyl)piperazin-1-yl)benzoic acid methyl ester (Compound 102)
   5-Chloro-3-(4-(3-chloropyridin-2-yl)piperazin-1-yl)benzo[d]isoxazole (Compound 111)
   (E)-(4-(3-Chloropyridin-2-yl)piperazin-1-yl) (2,5-dichlorophenyl)methanone oxime (Compound 112(E)) and its Z-isomer (Compound 112(Z))
   5-Chloro-6-(4-(5-chlorobenzo[d]isoxazol-3-yl)piperazin-1-yl)nicotinic acid (Compound 121)

   5-Chloro-6-(4-(5-(trifluoromethyl)benzo[d]isoxazol-3-yl)piperazin-1-yl)nicotinic acid (Compound 131)
   5-Chloro-6-(4-((2-chloro-5-(trifluoromethyl)phenyl)(hydroxyimino)methyl)piperazin-1-yl)nicotinic acid (Compound 132)
   3-(4-(Pyridin-2-yl)piperazin-1-yl)benzo[d]isoxazole (Compound 141)
   3-(4-(2-Chlorophenyl)piperazin-1-yl)benzo[d]isoxazole (Compound 151)
   3-(4-(2,3-Dimethylphenyl)piperazin-1-yl)benzo[d]isoxazole (Compound 161)
   3-(4-(2,4-Dichlorophenyl)piperazin-1-yl)benzo[d]isoxazole (Compound 171)
   3-(4-(3-Chloropyridin-2-yl)piperazin-1-yl)benzo[d]isothiazole (Compound 181)
   5-Chloro-6-(8-(4-(trifluoromethyl)benzo[d]isoxazol-3-yl)-3,8-diazabicyclo[3,2,1]octan-3-yl)nicotinic acid (Compound 191) 5-Chloro-6-(8-((2-fluoro-6-
   (trifluoromethyl)phenyl(hydroxyimino)methyl)-3,8-diazabicyclo[3,2,1]octan-3-yl)nicotinic acid (Compound 192) 7-Chloro-3-(4-(2-chlorophenyl)piperazin-1-yl)benzo[d]isoxazole (Compound 201)
   (E)-(4-(2-Chlorophenyl)piperazin-1-yl)(2,3-dichlorophenyl)methanone oxime (Compound 202(E)) and its Z-isomer (Compound 202(Z))
   6-Chloro-3-(4-(2-chlorophenyl)piperazin-1-yl)benzo[d]isoxazole (Compound 211)
   (E)-(4-(2-Chlorophenyl)piperazin-1-yl)(2,4-dichlorophenyl)methanone oxime (Compound 212(E)) and its Z-isomer (Compound 212(Z))
   5-Chloro-6-(4-(4-chlorobenzo[d]isoxazol-3-yl)piperazin-1-yl)pyridin-3-yl)(morpholino)methanone (Compound 221)
   5-Chloro-6-(4-(4-chlorobenzo[d]isoxazol-3-yl)piperazin-1-yl)-N-(methylsulfonyl)nicotinamide (Compound 231)
   Methyl 5-chloro-6-(4-(4-(trifluoromethyl)benzo[d]isoxazol-3-yl)piperazin-1-yl)nicotinate (Compound 241)
   (5-Chloro-6-(4-(4-(trifluoromethyl)benzo[d]isoxazol-3-yl)piperazin-1-yl)pyridin-3-yl)methanol (Compound 251)
   3-(4-(3-Methylpyridin-2-yl)piperazin-1-yl)-4-(trifluoromethyl)benzo[d]isoxazole (Compound 261)
   4-Methoxy-3-(4-(2-chlorophenyl)piperazin-1-yl)benzo[d]isoxazole (Compound 271)
   1-(1-(4-Trifluoromethyl)benzo[d]isoxazol-3-yl)piperidin-4-yl)-1,3-dihydro-2H-benzo[d]imidazol-2-one (Compound 281)
   1-(1-((2-Fluoro-6-(trifluoromethyl)phenyl(hydroxyimino)methyl)piperidin-4-yl)-1,3-dihydro-2H-benzo[d]imidazol-2-one (Compound 282
   2- (2-Oxo-3-(1-(4-(trifluoromethyl)benzo[d]isoxazol-3-yl)piperidin-4-yl)-2,3-dihydro-1H-benzo[d]imidazol-1-yl)acetic acid tert-butyl ester (Compound 291)
   2- (2-Oxo-3-(1-(4-(trifluoromethyl)benzo[d]isoxazol-3-yl)piperidin-4-yl)-2,3-dihydro-1H-benzo[d]imidazol-1-yl) acetic acid (Compound 301)
   3-(4-(2-Chloropyridin-3-yl)piperazin-1-yl)benzo[d]isoxazole (Compound 311)
   3-(4-(3-Chloropyridin-2-yl)piperazin-1-yl)benzo[d]isoxazole (Compound 321)
   3-(4-(3,5-Dichlorophenyl)piperazin-1-yl)benzo[d]isoxazole (Compound 331)
   Methyl 5-chloro-6-(1-(4-(trifluoromethyl)benzo[d]isoxazol-3-yl)piperidin-4-yl)nicotinate (Compound 341)
   Methyl 5-chloro-6-(1-((2-fluoro-6-(trifluoromethyl)phenyl)(hydroxyimino)methyl)piperidin-4-yl)nicotinate (Compound 342)
   5-chloro-6-(1-(4-(trifluoromethyl)benzo[d]isoxazol-3-yl)piperidin-4-yl)nicotinic acid (Compound 351)
   3-(4-(2-Chlorophenyl)-3,6-dihydropyridin-1(2H)-yl)-4-(trifluoromethyl)benzo[d]isoxazole (Compound 361)
   E-(4-(2-Chlorophenyl)-3,6-dihydropyridin-1(2H)-yl)(2-fluoro-6-(trifluoromethyl)phenyl)methanone oxime (Compound 362(E)) and its Z-isomer (Compound 362(Z))
   Ethyl 5-Chloro-6-(2-oxo-4-(4-(trifluoromethyl)benzo[d]isoxazol-3-yl)piperazin-1-yl)nicotinate (Compound 371)
   Ethyl (Z)-5-chloro-6-(4-((2-fluoro-6-(trifluoromethyl)phenyl)(hydroxyimino)methyl)-2-oxopiperazin-1-yl)nicotinate (Compound 372(Z))
   5-Chloro-6-(2-oxo-4-(4-(trifluoromethyl)benzo[d]isoxazol-3-yl)piperazin-1-yl)nicotinic acid (Compound 381)
   Methyl 3-chloro-4-(1-(4-(trifluoromethyl)benzo[d]isoxazol-3-yl)-1,2,3,6-tetrahydropyridin-4-yl)benzoate (Compound 391)
   Methyl 3-chloro-4-(1-(4-(trifluoromethyl)benzo[d]isoxazol-3-yl)-1,2,3,6-tetrahydropyridin-4-yl)benzoic acid (Compound 401)
   3-(4-(2-Chlorophenyl)piperazin-1-yl)-4-iodobenzo[d]isoxazole (Compound 411)
   (E)-(4-(2-Chlorophenyl)piperazin-1-yl)(2-fluoro-6-iodophenyl)methanone oxime (Compound 412(E)) and its Z-isomer (Compound 412(Z))
   3-(4-(2-Chlorophenyl)piperazin-1-yl)-4-cyanobenzo[d]isoxazole (Compound 421)
   3-(4-(2-Chlorophenyl)piperazin-1-yl)benzo[d]isoxazole-4-carboxyamide (Compound 431)

### Production Example 2

### Synthesis of 3-chloro-4-(4-(4-trifluoromethylbenzo[d]isoxazol-3-yl)piperazin-1-yl)benzoic acid (Compound 441)

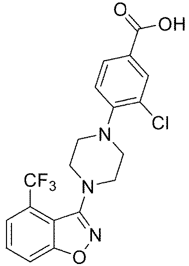

The title compound was obtained as described in Example 1 of WO2021/079962, except that 3-chloro-4-(piperazin-1-yl)benzoic acid ethyl ester hydrochloride was used instead of 5-chloro-6-(piperazin-1-yl)nicotinic acid hydrochloride.
¹H-NMR (CDCl₃, 400 MHz) δ8.12 (d, 1 H, J = 2.4 Hz), 7.99 (dd, 1 H, J = 1.6, 8.0 Hz), 7.75 (bd, 1 H, J = 8.0 Hz), 7.66 (dd, 1 H, J = 2.0, 8.0 Hz), 7.63 (t, 1 H, J = 8.0 Hz), 7.14 (d, 1 H, J = 8.8 Hz), 3.55 - 3.48 (m, 4 H), 3.42 - 3.32 (m, 4 H), LC-MS: r.t. 3.06 min., m/z 425.9 (M+1)

### Test Example 1

### 1. Action of Compound 011 on Urinary Protein Level in Rats with Puromycin Aminonucleoside-induced Kidney Injury

### Test Method

Eight-week-old S.D. rats (male, 8 rats) were intraperitoneally injected with 120 mg/kg of puromycin aminonucleoside (PAN) under anesthesia (day 0). PAN damages glomerular basement membrane cells (podocytes) and induces glomerular injury in rats. The PAN-treated rats were focal segmental glomerulosclerosis model rats and minimal change nephrotic syndrome model rats. The test compound (Compound 011) was mixed with food and administered (10 mg/kg/day) on or after the third day after the PAN injection. The rats were kept under a 12-hour light-dark cycle (lights on from 7:00 am to 7:00 pm, and lights off from 7:00 pm to 7:00 am the next morning), and food and water were provided ad libitum (PAN + 011 group). Urine was collected on day 0, i.e., the day of PAN administration, and on days 3, 10, 20, and 27 after PAN administration. Urine was collected by placing the rats in metabolic cages for 17 hours (5:00 pm to 10:00 am the next morning).

Separately, as controls, a group without PAN treatment and without test compound administration (control group, male, 3 animals), a group without PAN treatment but with test compound administration (control + 011 group, male, 5 animals), and a group with PAN treatment but without test compound administration (PAN group, male, 9 animals) were also tested in the same manner.

### Measurement Method

The urinary protein levels were measured by using the Bradford method.

The serum albumin levels were measured by using an improved BCP (bromocresol purple) method. In the PAN group, the number of serum samples was 8 because one animal from which a serum sample was unable to be collected was excluded.

On day 27 after PAN administration, the left kidney of each rat was removed, the size was visually observed, and the weight was measured. The number of left kidney samples of the control group was 3 because the left kidneys of three animals in this group were all removed and weighed. The number of left kidney samples of the control + 011 group was 4 because the left kidneys of 4 of the 5 animals in this group were removed and weighed. The number of left kidney samples of the PAN group was 7 because the left kidneys of 7 of the 9 animals in this group were removed and weighed. The number of left kidney samples of the PAN + 011 group was 7 because the left kidneys of 7 of the 8 animals in this group were removed and weighed.

### Test Results

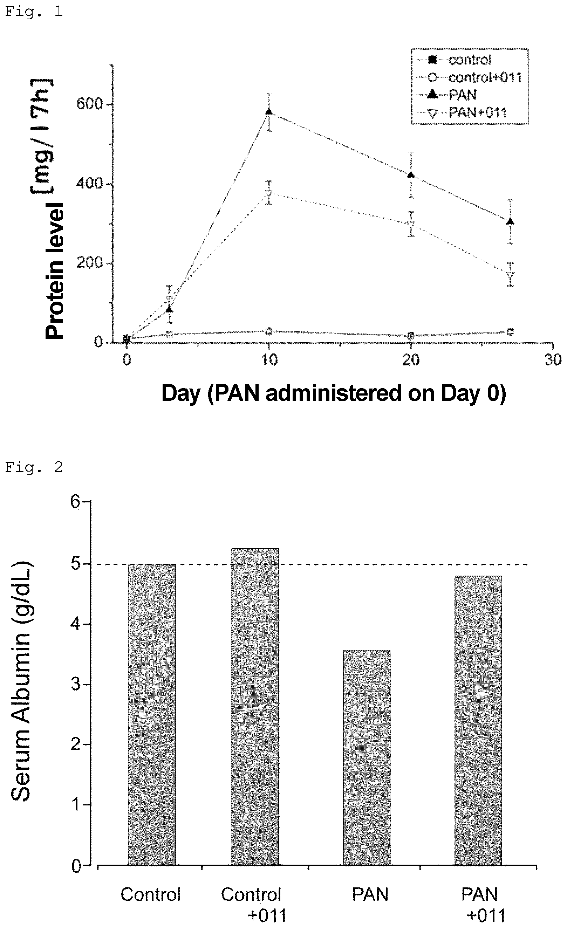

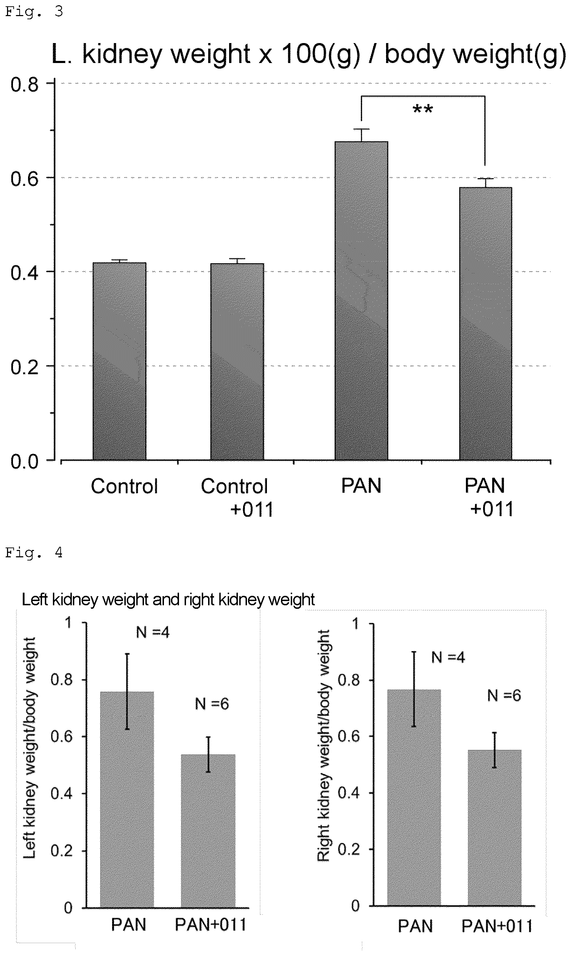

Fig. 1 shows the results of the measurement of the urinary protein levels, Fig. 2 shows the results of the measurement of the serum albumin levels (day 27), and Fig. 3 shows the results of the measurement of the left kidney weights.

No difference was observed in urinary protein levels between the control group and the control + 011 group. However, the urinary protein levels of the PAN + 011 group were 380 lower on day 10 and 480 lower on day 27, compared with those of the PAN group (Fig. 1). The test compound, i.e., Compound 011, clearly suppressed high urinary protein levels induced by PAN treatment.

The PAN group showed decreased serum albumin levels, whereby nephrotic syndrome-like conditions were confirmed. In contrast, the PAN + 011 group showed almost no PAN-treatment-induced decrease in serum albumin levels (Fig. 2). The test compound, i.e., Compound 011, clearly suppressed a PAN-treatment-induced decrease in serum albumin levels (** p<0.01).

In the PAN group, the kidneys were enlarged according to visual observation compared with those of the control group, and their masses were also clearly increased. The PAN + 011 group clearly showed a smaller increase in kidney weights.

### Test Example 2

### Action of Compound 011 on Diabetic Nephropathy Model Mice

### Test Method

Db/db mice, which are type 2 diabetes model mice, exhibit significant obesity and hyperglycemia due to overeating, and gradually develop high urinary protein levels due to glomerular hypertrophy and glomerular injury. Thus, these mice can be regarded as diabetic nephropathy model mice. The test compound (Compound 011) was mixed with food and administered for 8 weeks to 9-week-old db/db mice (10 mg/kg/day; diabetic nephropathy + 011 group), and the results were compared with the results of db/db mice that were treated in the same manner, except that the test compound was not administered (diabetic nephropathy group), whereby the effect of the test compound for suppressing urinary protein on the diabetic nephropathy model was studied. Other groups (a control group and a control + 011 group) were also prepared in the same manner, except that the db/db mice were replaced with 9-week-old db/+ mice (non-diabetic mice); these groups were groups with and without test compound administration. For these four groups, 6 male mice were used per group. The mice were kept under a 12-hour light-dark cycle (lights on from 7:00 am to 7:00 pm, and lights off from 7:00 pm to 7:00 am the next morning), and food and water were provided ad libitum. The urine samples were collected at the start of the test (9 weeks old), after 4 weeks (13 weeks old), and after 8 weeks (17 weeks old), and the urinary protein levels were measured according to the Bradford method.

### Test Example 3

### 2. Action of Compound 011 on Urinary Protein Level in Rats with Puromycin Aminonucleoside-induced Kidney Injury

The action of Compound 011 on PAN-treated rats with more severe symptoms for a longer period of time than in Test Example 1 was studied.

### Test Method

Eight-week-old S.D. rats (male, 8 rats) were intraperitoneally injected with 120 mg/kg of puromycin aminonucleoside (PAN) under anesthesia. Then, one month later, PAN was again injected intraperitoneally at 60 mg/kg. PAN creates focal segmental glomerulosclerosis model rats by disrupting glomerular basement membrane cells (podocytes) (Kim, J Mol Med, 2018, 96,631-644). Furthermore, these model rats are known to develop chronic kidney damage accompanied by lymphocyte and macrophage infiltration (Ornellas, Sci Rep, 2019, 9:19604). The test compound (Compound 011) was mixed with food and administered (10 mg/kg/day) on or after the third day after the PAN injection. The rats were kept under a 12-hour light-dark cycle (lights on from 7:00 am to 7:00 pm, and lights off from 7:00 pm to 7:00 am the next morning), and food and water were provided ad libitum (PAN + 011 group). Chronic proteinuria may sometimes occur after the first administration of PAN without a second administration of PAN. Therefore, if a rat that received first administration of PAN showed a sufficiently high value of proteinuria, the second administration of PAN was not given to that rat. The results of rats that did not receive second administration of PAN were also included in the statistics.

### Measurement Method

Two months after the first administration of PAN, the kidneys of the rats were removed, their sizes were visually observed, and their weights were measured. The number of kidney samples was 4 for the PAN group and was 6 for the PAN + 011 group. Subsequently, the kidneys were fixed with 4% paraformaldehyde/PBS and then paraffin-embedded, and sections were prepared. The sections were treated with PAS (periodic acid-Schiff) staining, and PAS-stained positive areas (inflammatory cell areas) were evaluated.

To extract RNA, a portion of tissue around the surface layer of the kidney was quickly frozen to -80°C. cDNA was produced from RNA extracted from the tissue by reverse transcriptase, and gene expression was evaluated by real-time PCR. TB Green Premix Ex Taq II (Takara Bio Inc.) was used as a reagent for the reaction, and a QuantStudio 5 system (Thermo Fisher) was used for the analysis.

### Test Results

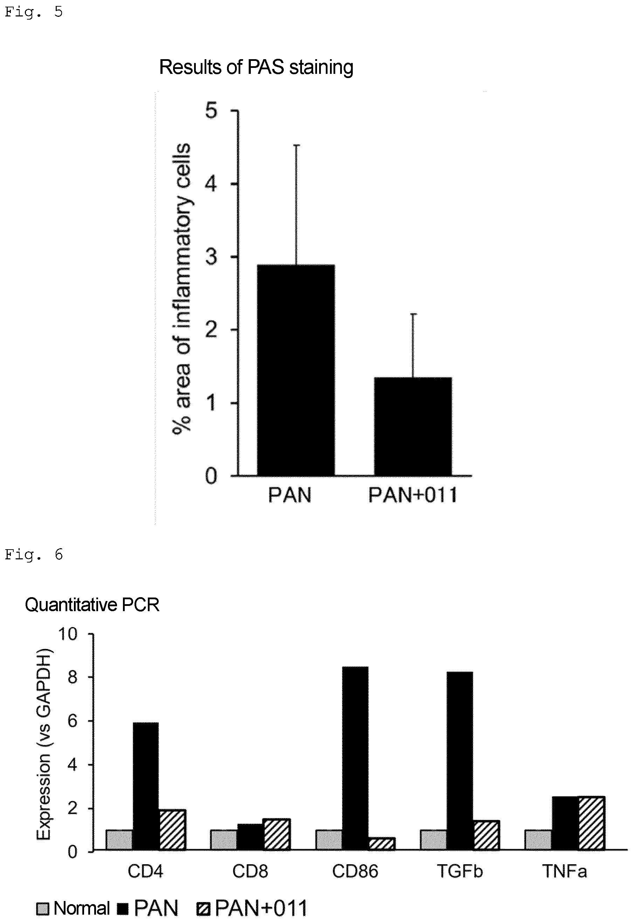

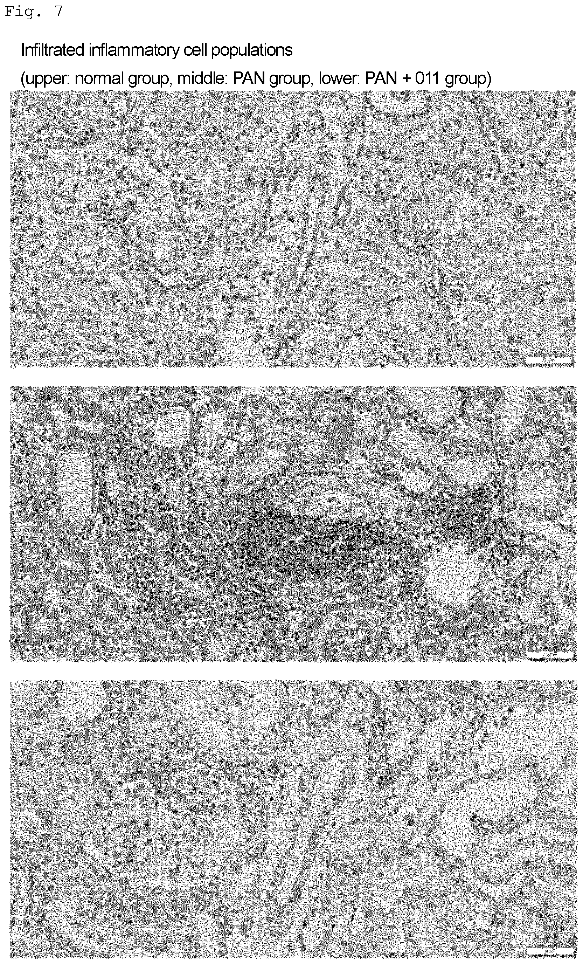

Fig. 4 shows the results of the measurement of left and right kidney weights, and Fig. 5 shows the results of PAS staining analysis. Fig. 6 shows the results of quantitative PCR of expressed genes collected from the kidneys. Fig. 7 shows infiltrated inflammatory cell populations.

In the PAN + 011 group, the left kidney weight per rat body weight was decreased by 29% and the right kidney weight by 28% after 2 months compared with the left and right kidney weights of the PAN group (Fig. 4). The test compound, i.e., Compound 011, significantly suppressed a PAN-treatment-induced increase in left and right kidney weights (ANOVA test, * p<0.03).

Two months after the PAN administration, tertiary lymphoid tissue-like structures (Sato, Yanagida, The Japanese Journal of Nephrology, 2023, 65, 43-47) were formed in several sites at the border area between the cortex and medulla of the kidney tissue due to the infiltration of inflammatory cell populations. The area was stained deep purple with PAS staining. In contrast, in the PAN + 011 group, the area of tertiary lymphoid tissue-like structures composed of inflammatory cell populations was greatly decreased (Figs. 5 and 7). The test compound, i.e., compound 011, suppressed the formation of tertiary lymphoid tissue-like structures formed by the infiltration of inflammatory cell populations formed over time after PAN treatment by 50% or more on average.

Gene expression in the tissue removed from the rat kidneys two months after the PAN administration was evaluated by using quantitative PCR, the results of which showed an increase in CD4, i.e., a type of lymphocyte and a T-cell marker, CD86, i.e., a macrophage marker, and TGF-β, i.e., a factor involved in cell growth and differentiation (Fig. 6). In contrast, the expression levels of CD4, CD86, and TGF-β, which were increased due to PAN, were significantly decreased in the PAN + 011 group, to which Compound 011 was given (Fig. 6). In the graph shown in Fig. 6, the expression levels of the target genes in each individual are shown as relative values, with the expression level of GAPDH (glyceraldehyde-3-phosphate dehydrogenase) in each individual taken as 1.

## Claims

1. A pharmaceutical composition for preventing and/or treating a kidney disease, comprising a compound represented by formula (1), a salt thereof, or a prodrug thereof: 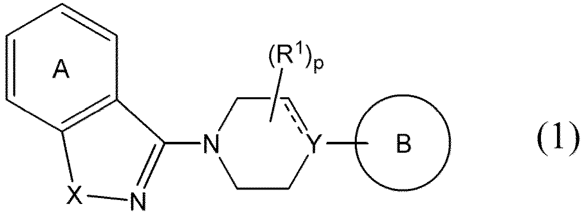 wherein
A is an optionally substituted benzene ring;
B is an optionally substituted aryl or an optionally substituted heteroaryl;
X is an oxygen atom or a sulfur atom;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

2. The pharmaceutical composition according to claim 1, wherein in formula (1),
B is an optionally substituted monocyclic aryl or an optionally substituted monocyclic or bicyclic nitrogen-containing heteroaryl.

3. The pharmaceutical composition according to claim 1 or 2, wherein in formula (1),
A is a benzene ring optionally substituted with at least one substituent selected from the group consisting of the following A-1 to A-16:
A-1) halogen,
A-2) hydroxyl,
A-3) nitro,
A-4) cyano,
A-5) carboxyl,
A-6) optionally substituted amino,
A-7) optionally substituted cyclic amino,
A-8) optionally substituted lower alkyl,
A-9) optionally substituted lower alkoxy,
A-10) lower alkoxycarbonyl,
A-11) lower alkylsulfonyl,
A-12) carbamoyl optionally substituted with lower alkyl or lower alkylsulfonyl,
A-13) optionally substituted cyclic aminocarbonyl,
A-14) sulfamoyl optionally substituted with lower alkyl,
A-15) optionally substituted cyclic aminosulfonyl, and
A-16) tetrazolyl.

4. The pharmaceutical composition according to any one of claims 1 to 3, wherein in formula (1),
B is a monocyclic aryl or a monocyclic or bicyclic heteroaryl, the monocyclic aryl is optionally substituted with at least one substituent selected from the group consisting of the following B-1 to B-16, and
the monocyclic or bicyclic heteroaryl is optionally substituted with at least one substituent selected from the group consisting of the following B-1 to B-17:
B-1) halogen,
B-2) hydroxyl,
B-3) nitro,
B-4) cyano,
B-5) carboxyl
B-6) optionally substituted amino,
B-7) optionally substituted cyclic amino,
B-8) optionally substituted lower alkyl,
B-9) optionally substituted lower alkoxy,
B-10) lower alkoxycarbonyl,
B-11) lower alkylsulfonyl,
B-12) carbamoyl optionally substituted with lower alkyl or lower alkylsulfonyl,
B-13) optionally substituted cyclic aminocarbonyl,
B-14) sulfamoyl optionally substituted with lower alkyl,
B-15) optionally substituted cyclic aminosulfonyl,
B-16) tetrazolyl, and
B-17) oxo.

5. The pharmaceutical composition according to any one of claims 1 to 4, wherein in formula (1), the benzisoxazole or benzisothiazole skeleton is substituted at the 4-position.

6. The pharmaceutical composition according to any one of claims 1 to 3, wherein in formula (1),
B is substituted pyridyl or substituted phenyl, and at least one of the carbon atoms in ortho-positions with respect to the Y-bound carbon atom on the pyridine or benzene ring is substituted.

7. The pharmaceutical composition according to any one of claims 1 to 4, wherein in formula (1),
A is a benzene ring optionally substituted with at least one substituent selected from the group consisting of halogen, lower alkoxy, and optionally halogen-substituted lower alkyl;
B is pyridyl or phenyl each optionally substituted with at least one substituent selected from the group consisting of the following B-1, B-5, B-8, B-10, B-12, and B-13:
B-1) halogen,
B-5) carboxyl,
B-8) optionally substituted lower alkyl,
B-10) lower alkoxycarbonyl,
B-12) carbamoyl optionally substituted with lower alkyl or lower alkylsulfonyl, and
B-13) optionally substituted cyclic aminocarbonyl; and
each R¹ independently represents C₁-C₃ alkyl, or
two R¹s are bound to each other to form a methylene group, a dimethylene group, or a trimethylene group, or
(R¹)ₚ is oxo.

8. The pharmaceutical composition according to any one of claims 1 to 4 and 7, wherein the compound represented by formula (1) is a compound represented by formula (1A): 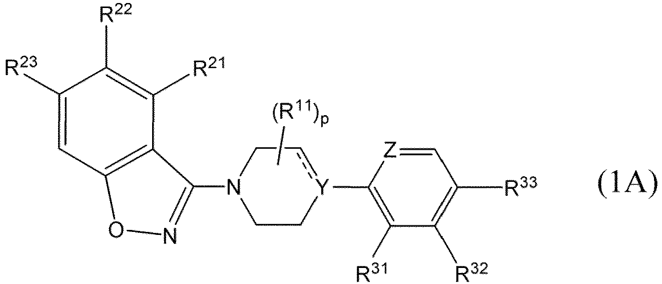 wherein
Z is a nitrogen atom or CH;
Y is a nitrogen atom or a carbon atom;
- - - - - - of - - - - -Y is a single or double bond when Y is a carbon atom, or - - - - - - of - - - - -Y is a single bond when Y is a nitrogen atom;
each R¹¹ independently represents methyl or ethyl, or
two R¹¹s may be bound to each other to form a crosslinked structure by methylene, dimethylene, or trimethylene;
p is 0, 1, or 2; or
(R¹¹)ₚ is oxo;
R²¹, R²², and R²³ independently represent a hydrogen atom, halogen, carbamoyl, or trifluoromethyl; and
R³¹, R³², and R³³ independently represent a hydrogen atom, halogen, halogen-substituted lower alkyl, methyl, carboxyl, lower alkoxycarbonyl, monomethylaminocarbonyl, or
dimethylaminocarbonyl.

9. The pharmaceutical composition according to claim 8, wherein in formula (1A),
R²¹ is a chlorine atom or trifluoromethyl,
R²² and R²³ are each a hydrogen atom,
R³¹ is a chlorine atom,
R³² is a hydrogen atom, and
R³³ is a hydrogen atom, carboxyl, or lower alkoxycarbonyl.

10. The pharmaceutical composition according to any one of claims 1 to 4, wherein the compound is Compound 011, Compound 021, Compound 031, Compound 041, Compound 061, Compound 071, Compound 081, Compound 091, Compound 101, Compound 111, Compound 121, Compound 131, Compound 141, Compound 151, Compound 161, Compound 171, Compound 191, Compound 221, Compound 281, Compound 311, Compound 321, Compound 331, Compound 341, Compound 351, Compound 361, Compound 371, Compound 381, Compound 391, Compound 401, Compound 431, or Compound 441, each represented by any of the following structures:

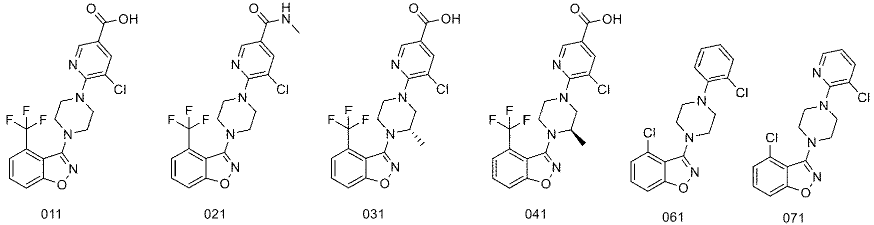

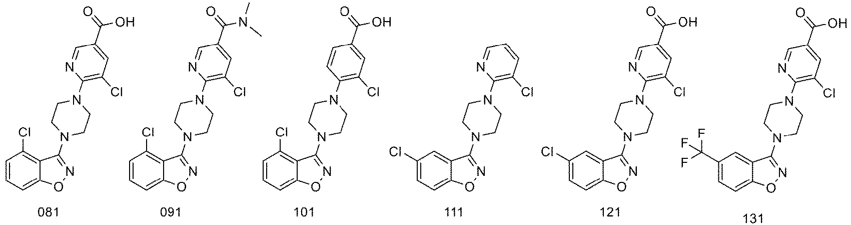

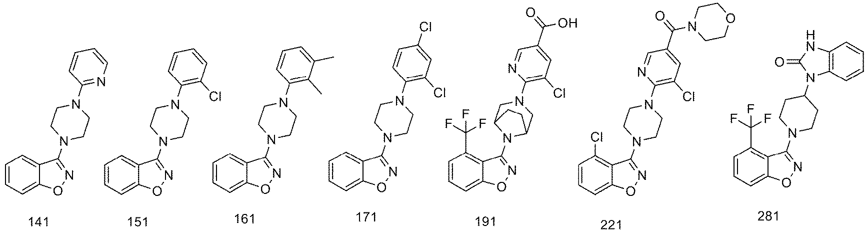

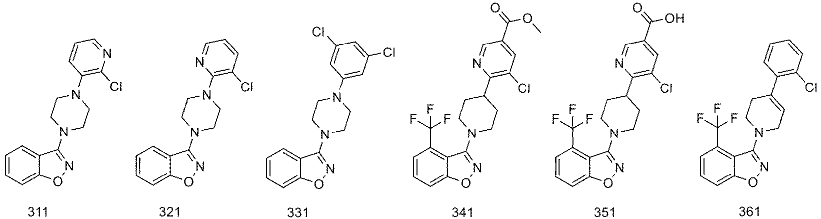

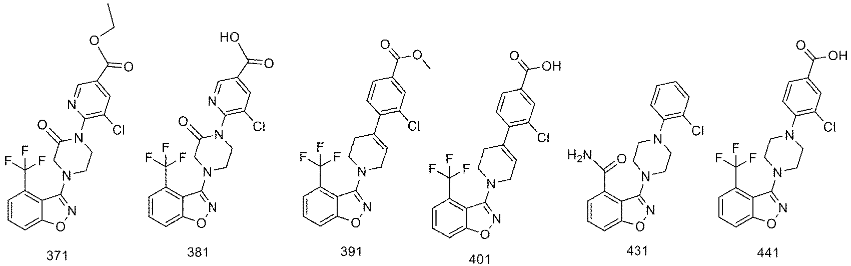

11. A pharmaceutical composition for preventing and/or treating a kidney disease, comprising a compound represented by formula (2), a salt thereof, or a prodrug thereof: 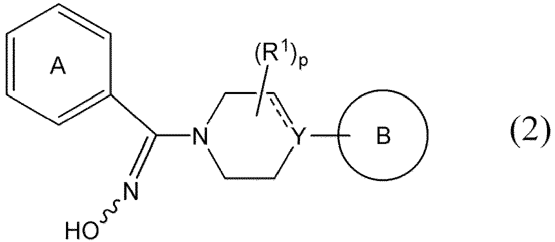 wherein
A is an optionally substituted benzene ring;
B is an optionally substituted aryl or an optionally substituted heteroaryl;
Y is a nitrogen atom or a carbon atom;
- - - - - - of - - - - -Y is a single or double bond when Y is a carbon atom, or
- - - - - - of - - - - -Y is a single bond when Y is a nitrogen atom;
each R¹ independently represents lower alkyl, or
two R¹s may be bound to each other to form a spiro ring or a crosslinked structure, or
two R¹s may be bound to each other to form a saturated fused heterocycle together with nitrogen and carbon atoms constituting a ring containing Y;
p is 0, 1, or 2; or
(R¹)ₚ is oxo.

12. The pharmaceutical composition according to any one of claims 1 to 11, wherein the kidney disease is a glomerular disease.

13. The pharmaceutical composition according to claim 12, wherein the glomerular disease is chronic glomerulonephritis.

14. The pharmaceutical composition according to claim 12, wherein the glomerular disease is diabetic nephropathy.

15. The pharmaceutical composition according to any one of claims 12 to 14, wherein the glomerular disease is a disease accompanied by proteinuria.

16. A pharmaceutical composition according to any one of claims 1 to 15, which is for oral administration.
